# EUROPEAN PATENT APPLICATION

(11) **EP 2 671 891 A2**
(43) Date of publication of application: **11.12.2013**
(21) Application number: 13183424.4
(22) Date of filing: 24.06.2009
(51) Int. Cl.: C07K 16/22, A61K 39/395

(54) **Ang-2 inhibition to treat multiple sclerosis**

(30) Priority: 27.06.2008 US 76431 P
(62) Divisional of application: 09770965.3
(71) Applicant: Amgen Inc., California 91320 (US)
(72) Inventor: Kirchner, Jacqueline A., Seattle, WA 98125 (US); Mustelin, Tomas Mikael, Seattle, WA 98199 (US)
(74) Representative: Care, Alison

(57) **Abstract**

Methods and compositions are disclosed for treating multiple sclerosis in a human subject by administering a therapeutically effective amount of an 2XCon4(C) peptibody.

SEQUENCE LISTING

<110> AMGEN INC.
      Kirchner, Jacqueline A.
      Mustelin, Tomas Mikael

<120> ANG-2 INHIBITION TO TREAT MULTIPLE SCLEROSIS

<130> A-1438-WO-PCT

<140> to be assigned
<141> 2009-06-24

<150> US 61/076,431
<151> 2008-06-27

<160> 57

<170> PatentIn version 3.4

<210> 1
<211> 122
<212> PRT
<213> Homo sapiens

<400> 1

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Glu Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Ile Leu Thr Gly Tyr Gly Tyr Trp
            100                 105                 110

Gly Gln Gly Thr Leu Val Thr Val Ser Ser
        115                 120


<210> 2
<211> 122
<212> PRT
<213> Homo sapiens

<400>  2

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Gln Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Ile Leu Thr Gly Tyr Gly Tyr Trp
            100                 105                 110

Gly Gln Gly Thr Leu Val Thr Val Ser Ser
        115                 120


<210>  3
<211>  122
<212>  PRT
<213>  Homo sapiens

<400>  3

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys

                85                  90                  95

Ala Arg Asp Leu Leu Asp Tyr Asp Leu Leu Thr Gly Tyr Gly Tyr Trp
            100                 105                 110

Gly Gln Gly Thr Leu Val Thr Val Ser Ser
        115                 120

<210> 4
<211> 122
<212> PRT
<213> Homo sapiens

<400> 4

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Ile Tyr Thr Gly Tyr Gly Tyr Trp
            100                 105                 110

Gly Gln Gly Thr Leu Val Thr Val Ser Ser
        115                 120

<210> 5
<211> 122
<212> PRT
<213> Homo sapiens

<400> 5

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Ile Leu Thr Gly Tyr Gly Leu Trp
            100                 105                 110

Gly Gln Gly Thr Leu Val Thr Val Ser Ser
        115                 120

<210> 6
<211> 122
<212> PRT
<213> Homo sapiens

<400> 6

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Ile Leu Thr Gly Tyr Gly Met Trp
            100                 105                 110

Gly Gln Gly Thr Leu Val Thr Val Ser Ser
        115                 120

<210>  7
<211>  122
<212>  PRT
<213>  Homo sapiens

<400>  7

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Ile Trp Thr Gly Tyr Gly Tyr Trp
            100                 105                 110

Gly Gln Gly Thr Leu Val Thr Val Ser Ser
        115                 120


<210>  8
<211>  112
<212>  PRT
<213>  Homo sapiens

<400>  8

Asp Ile Val Met Thr Gln Ser Pro Leu Ser Leu Pro Val Thr Pro Gly
1               5                   10                  15

Glu Pro Ala Ser Ile Ser Cys Arg Ser Ile Gln Ser Leu Leu Gln Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110

<210>  9
<211>  112
<212>  PRT
<213>  Homo sapiens

<400>  9

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu Leu Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110

<210>  10
<211>  112
<212>  PRT
<213>  Homo sapiens

<400>  10

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

His Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110

<210>  11
<211>  112
<212>  PRT
<213>  Homo sapiens

<400>  11

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Val Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110

<210>  12
<211>  112
<212>  PRT
<213>  Homo sapiens

<400>  12

Asp Ile Val Met Thr Gln Ser Pro Leu Ser Leu Pro Val Thr Pro Gly

1               5                   10                  15

Glu Pro Ala Ser Ile Ser Cys Arg Ser Ser Gln Ser Leu Leu His Ser
            20                  25                  30

Asn Gly Tyr Asn Phe Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110

<210>  13
<211>  112
<212>  PRT
<213>  Homo sapiens

<400>  13

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Met Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110

<210>  14

<211>  112
<212>  PRT
<213>  Homo sapiens

<400>  14

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Ala Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110


<210>  15
<211>  112
<212>  PRT
<213>  Homo sapiens

<400>  15

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asp Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110

<210> 16
<211> 112
<212> PRT
<213> Homo sapiens

<400> 16

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Thr His Trp Pro Pro Thr Phe Gly Gln Gly Thr Lys Leu Glu Ile Lys
            100                 105                 110

<210> 17
<211> 112
<212> PRT
<213> Homo sapiens

<400> 17

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Val
                85                  90                  95

Thr His Trp Pro Pro Thr Phe Gly Gln Gly Thr Lys Leu Glu Ile Lys
            100                 105                 110

<210>  18
<211>  5
<212>  PRT
<213>  Homo sapiens

<400>  18

Ser Tyr Gly Met His
1               5

<210>  19
<211>  17
<212>  PRT
<213>  Homo sapiens

<400>  19

Tyr Ile Ser Ser Ser Gly Ser Thr Ile Tyr Tyr Ala Asp Ser Val Lys
1               5                   10                  15

Gly

<210>  20
<211>  17
<212>  PRT
<213>  Homo sapiens

<400>  20

<210>  21
<211>  17
<212>  PRT
<213>  Homo sapiens

<400>  21

<210> 22
<211> 13
<212> PRT
<213> Homo sapiens

<400> 22

Asp Leu Leu Asp Tyr Asp Ile Tyr Thr Gly Tyr Gly Tyr
1               5                   10

<210> 23
<211> 13
<212> PRT
<213> Homo sapiens

<400> 23

Asp Leu Leu Asp Tyr Asp Ile Trp Thr Gly Tyr Gly Tyr
1               5                   10

<210> 24
<211> 13
<212> PRT
<213> Homo sapiens

<400> 24

Asp Leu Leu Asp Tyr Asp Leu Leu Thr Gly Tyr Gly Tyr
1               5                   10

<210> 25
<211> 13
<212> PRT
<213> Homo sapiens

<400> 25

Asp Leu Leu Asp Tyr Asp Ile Leu Thr Gly Met Gly Tyr
1               5                   10

<210> 26
<211> 13
<212> PRT
<213> Homo sapiens

<400> 26

Asp Leu Leu Asp Tyr Asp Ile Leu Thr Gly Leu Gly Tyr
1               5                   10

<210> 27
<211> 13

<212>  PRT
<213>  Homo sapiens

<400>  27

Asp Leu Leu Asp Tyr Asp Ile Leu Thr Gly Tyr Gly Tyr
1               5                   10


<210>  28
<211>  16
<212>  PRT
<213>  Homo sapiens

<400>  28

Arg Ser Ser Gln Ser Leu Leu His Ser Asn Gly Tyr Asn Phe Leu Asp
1               5                   10                  15


<210>  29
<211>  16
<212>  PRT
<213>  Homo sapiens

<400>  29

Arg Ser Ser Gln Ser Leu Leu His Ser Asn Gly Tyr Asn Tyr Leu Asp
1               5                   10                  15


<210>  30
<211>  16
<212>  PRT
<213>  Homo sapiens

<400>  30

Arg Ser Ser Gln Ser Leu Leu His Ser Val Gly Tyr Asn Tyr Leu Asp
1               5                   10                  15


<210>  31
<211>  16
<212>  PRT
<213>  Homo sapiens

<400>  31

Arg Ser Ser Gln Ser Leu Leu His Ser Asn Gly Tyr Asn Met Leu Asp
1               5                   10                  15


<210>  32
<211>  16
<212>  PRT
<213>  Homo sapiens

<400>  32

Arg Ser Ser Gln Ser Leu Leu His Ser His Gly Tyr Asn Tyr Leu Asp
1               5                   10                  15

<210>  33
<211>  16
<212>  PRT
<213>  Homo sapiens

<400>  33

Arg Ser Ser Gln Ser Leu Leu Leu Ser Asn Gly Tyr Asn Tyr Leu Asp
1               5                   10                  15


<210>  34
<211>  16
<212>  PRT
<213>  Homo sapiens

<400>  34

Arg Ser Ile Gln Ser Leu Leu Gln Ser Asn Gly Tyr Asn Tyr Leu Asp
1               5                   10                  15


<210>  35
<211>  7
<212>  PRT
<213>  Homo sapiens

<400>  35

Leu Gly Ser Asn Arg Ala Ser
1               5


<210>  36
<211>  7
<212>  PRT
<213>  Homo sapiens

<400>  36

Leu Gly Ser Asp Arg Ala Ser
1               5


<210>  37
<211>  7
<212>  PRT
<213>  Homo sapiens

<400>  37

Ala Gly Ser Asn Arg Ala Ser
1               5


<210>  38
<211>  9
<212>  PRT
<213>  Homo sapiens

<400>  38

Met Gln Gly Thr His Trp Pro Pro Thr
1               5

<210>  39
<211>  9
<212>  PRT
<213>  Homo sapiens

<400>  39

Met Gln Val Thr His Trp Pro Pro Thr
1               5

<210>  40
<211>  9
<212>  PRT
<213>  Homo sapiens

<400>  40

Met Gln Ala Thr His Trp Pro Pro Thr
1               5

<210>  41
<211>  122
<212>  PRT
<213>  Homo sapiens

<400>  41

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Ile Leu Thr Gly Tyr Gly Tyr Trp
            100                 105                 110

Gly Gln Gly Thr Leu Val Thr Val Ser Ser
        115                 120

<210>  42
<211>  112
<212>  PRT
<213>  Homo sapiens

<400>  42

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Phe Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Thr His Trp Pro Pro Thr Phe Gly Gln Gly Thr Lys Leu Glu Ile Lys
            100                 105                 110


<210>  43
<211>  112
<212>  PRT
<213>  Homo sapiens

<400>  43

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Gly
                85                  90                  95

Leu Gln Thr Pro Pro Thr Phe Gly Gln Gly Thr Lys Leu Glu Ile Lys
            100                 105                 110

<210>  44
<211>  22
<212>  PRT
<213>  Homo sapiens

<400>  44

Thr Asn Phe Met Pro Met Asp Asp Leu Glu Gln Arg Leu Tyr Glu Gln
1               5                   10                  15

Phe Ile Leu Gln Gln Gly
            20

<210>  45
<211>  22
<212>  PRT
<213>  Homo sapiens

<400>  45

Gln Lys Phe Gln Pro Leu Asp Glu Leu Glu Gln Thr Leu Tyr Glu Gln
1               5                   10                  15

Phe Met Leu Gln Gln Ala
            20

<210>  46
<211>  1124
<212>  PRT
<213>  Homo sapiens

<400>  46

Met Asp Ser Leu Ala Ser Leu Val Leu Cys Gly Val Ser Leu Leu Leu
1               5                   10                  15

Ser Gly Thr Val Glu Gly Ala Met Asp Leu Ile Leu Ile Asn Ser Leu
            20                  25                  30

Pro Leu Val Ser Asp Ala Glu Thr Ser Leu Thr Cys Ile Ala Ser Gly
        35                  40                  45

Trp Arg Pro His Glu Pro Ile Thr Ile Gly Arg Asp Phe Glu Ala Leu
    50                  55                  60

Met Asn Gln His Gln Asp Pro Leu Glu Val Thr Gln Asp Val Thr Arg

65                  70                  75                  80

Glu Trp Ala Lys Lys Val Val Trp Lys Arg Glu Lys Ala Ser Lys Ile
                85                  90                  95

Asn Gly Ala Tyr Phe Cys Glu Gly Arg Val Arg Gly Glu Ala Ile Arg
            100                 105                 110

Ile Arg Thr Met Lys Met Arg Gln Gln Ala Ser Phe Leu Pro Ala Thr
        115                 120                 125

Leu Thr Met Thr Val Asp Lys Gly Asp Asn Val Asn Ile Ser Phe Lys
    130                 135                 140

Lys Val Leu Ile Lys Glu Glu Asp Ala Val Ile Tyr Lys Asn Gly Ser
145                 150                 155                 160

Phe Ile His Ser Val Pro Arg His Glu Val Pro Asp Ile Leu Glu Val
                165                 170                 175

His Leu Pro His Ala Gln Pro Gln Asp Ala Gly Val Tyr Ser Ala Arg
            180                 185                 190

Tyr Ile Gly Gly Asn Leu Phe Thr Ser Ala Phe Thr Arg Leu Ile Val
        195                 200                 205

Arg Arg Cys Glu Ala Gln Lys Trp Gly Pro Glu Cys Asn His Leu Cys
    210                 215                 220

Thr Ala Cys Met Asn Asn Gly Val Cys His Glu Asp Thr Gly Glu Cys
225                 230                 235                 240

Ile Cys Pro Pro Gly Phe Met Gly Arg Thr Cys Glu Lys Ala Cys Glu
                245                 250                 255

Leu His Thr Phe Gly Arg Thr Cys Lys Glu Arg Cys Ser Gly Gln Glu
            260                 265                 270

Gly Cys Lys Ser Tyr Val Phe Cys Leu Pro Asp Pro Tyr Gly Cys Ser
        275                 280                 285

Cys Ala Thr Gly Trp Lys Gly Leu Gln Cys Asn Glu Ala Cys His Pro
    290                 295                 300

Gly Phe Tyr Gly Pro Asp Cys Lys Leu Arg Cys Ser Cys Asn Asn Gly
305                 310                 315                 320

Glu Met Cys Asp Arg Phe Gln Gly Cys Leu Cys Ser Pro Gly Trp Gln
                325                 330                 335

Gly Leu Gln Cys Glu Arg Glu Gly Ile Pro Arg Met Thr Pro Lys Ile
            340                 345                 350

Val Asp Leu Pro Asp His Ile Glu Val Asn Ser Gly Lys Phe Asn Pro
        355                 360                 365

Ile Cys Lys Ala Ser Gly Trp Pro Leu Pro Thr Asn Glu Glu Met Thr
    370                 375                 380

Leu Val Lys Pro Asp Gly Thr Val Leu His Pro Lys Asp Phe Asn His
385                 390                 395                 400

Thr Asp His Phe Ser Val Ala Ile Phe Thr Ile His Arg Ile Leu Pro
                405                 410                 415

Pro Asp Ser Gly Val Trp Val Cys Ser Val Asn Thr Val Ala Gly Met
            420                 425                 430

Val Glu Lys Pro Phe Asn Ile Ser Val Lys Val Leu Pro Lys Pro Leu
        435                 440                 445

Asn Ala Pro Asn Val Ile Asp Thr Gly His Asn Phe Ala Val Ile Asn
    450                 455                 460

Ile Ser Ser Glu Pro Tyr Phe Gly Asp Gly Pro Ile Lys Ser Lys Lys
465                 470                 475                 480

Leu Leu Tyr Lys Pro Val Asn His Tyr Glu Ala Trp Gln His Ile Gln
                485                 490                 495

Val Thr Asn Glu Ile Val Thr Leu Asn Tyr Leu Glu Pro Arg Thr Glu
            500                 505                 510

Tyr Glu Leu Cys Val Gln Leu Val Arg Arg Gly Glu Gly Gly Glu Gly
        515                 520                 525

His Pro Gly Pro Val Arg Arg Phe Thr Thr Ala Ser Ile Gly Leu Pro
    530                 535                 540

Pro Pro Arg Gly Leu Asn Leu Leu Pro Lys Ser Gln Thr Thr Leu Asn
545                 550                 555                 560

Leu Thr Trp Gln Pro Ile Phe Pro Ser Ser Glu Asp Asp Phe Tyr Val
                565                 570                 575

Glu Val Glu Arg Arg Ser Val Gln Lys Ser Asp Gln Gln Asn Ile Lys
        580                 585                 590

Val Pro Gly Asn Leu Thr Ser Val Leu Leu Asn Asn Leu His Pro Arg
    595                 600                 605

Glu Gln Tyr Val Val Arg Ala Arg Val Asn Thr Lys Ala Gln Gly Glu
    610                 615                 620

Trp Ser Glu Asp Leu Thr Ala Trp Thr Leu Ser Asp Ile Leu Pro Pro
625                 630                 635                 640

Gln Pro Glu Asn Ile Lys Ile Ser Asn Ile Thr His Ser Ser Ala Val
                645                 650                 655

Ile Ser Trp Thr Ile Leu Asp Gly Tyr Ser Ile Ser Ser Ile Thr Ile
        660                 665                 670

Arg Tyr Lys Val Gln Gly Lys Asn Glu Asp Gln His Val Asp Val Lys
    675                 680                 685

Ile Lys Asn Ala Thr Ile Thr Gln Tyr Gln Leu Lys Gly Leu Glu Pro
    690                 695                 700

Glu Thr Ala Tyr Gln Val Asp Ile Phe Ala Glu Asn Asn Ile Gly Ser
705                 710                 715                 720

Ser Asn Pro Ala Phe Ser His Glu Leu Val Thr Leu Pro Glu Ser Gln
                725                 730                 735

Ala Pro Ala Asp Leu Gly Gly Gly Lys Met Leu Leu Ile Ala Ile Leu
        740                 745                 750

Gly Ser Ala Gly Met Thr Cys Leu Thr Val Leu Leu Ala Phe Leu Ile
    755                 760                 765

Ile Leu Gln Leu Lys Arg Ala Asn Val Gln Arg Arg Met Ala Gln Ala
    770                 775                 780

Phe Gln Asn Val Arg Glu Glu Pro Ala Val Gln Phe Asn Ser Gly Thr
785                 790                 795                 800

Leu Ala Leu Asn Arg Lys Val Lys Asn Asn Pro Asp Pro Thr Ile Tyr
                805                 810                 815

Pro Val Leu Asp Trp Asn Asp Ile Lys Phe Gln Asp Val Ile Gly Glu
        820                 825                 830

Gly Asn Phe Gly Gln Val Leu Lys Ala Arg Ile Lys Lys Asp Gly Leu
        835                 840                 845

Arg Met Asp Ala Ala Ile Lys Arg Met Lys Glu Tyr Ala Ser Lys Asp
    850                 855                 860

Asp His Arg Asp Phe Ala Gly Glu Leu Glu Val Leu Cys Lys Leu Gly
865                 870                 875                 880

His His Pro Asn Ile Ile Asn Leu Leu Gly Ala Cys Glu His Arg Gly
                885                 890                 895

Tyr Leu Tyr Leu Ala Ile Glu Tyr Ala Pro His Gly Asn Leu Leu Asp
            900                 905                 910

Phe Leu Arg Lys Ser Arg Val Leu Glu Thr Asp Pro Ala Phe Ala Ile
        915                 920                 925

Ala Asn Ser Thr Ala Ser Thr Leu Ser Ser Gln Gln Leu Leu His Phe
    930                 935                 940

Ala Ala Asp Val Ala Arg Gly Met Asp Tyr Leu Ser Gln Lys Gln Phe
945                 950                 955                 960

Ile His Arg Asp Leu Ala Ala Arg Asn Ile Leu Val Gly Glu Asn Tyr
                965                 970                 975

Val Ala Lys Ile Ala Asp Phe Gly Leu Ser Arg Gly Gln Glu Val Tyr
            980                 985                 990

Val Lys Lys Thr Met Gly Arg Leu Pro Val Arg Trp Met Ala Ile Glu
        995                 1000                1005

Ser Leu Asn Tyr Ser Val Tyr Thr Thr Asn Ser Asp Val Trp Ser
    1010                1015                1020

Tyr Gly Val Leu Leu Trp Glu Ile Val Ser Leu Gly Gly Thr Pro
    1025                1030                1035

Tyr Cys Gly Met Thr Cys Ala Glu Leu Tyr Glu Lys Leu Pro Gln
    1040                1045                1050

Gly Tyr Arg Leu Glu Lys Pro Leu Asn Cys Asp Asp Glu Val Tyr
    1055                1060                1065

Asp Leu Met Arg Gln Cys Trp Arg Glu Lys Pro Tyr Glu Arg Pro

1070                 1075                 1080

Ser Phe  Ala Gln Ile Leu Val  Ser Leu Asn Arg Met  Leu Glu Glu
    1085                 1090                 1095

Arg Lys  Thr Tyr Val Asn Thr  Thr Leu Tyr Glu Lys  Phe Thr Tyr
    1100                 1105                 1110

Ala Gly  Ile Asp Cys Ser Ala  Glu Glu Ala Ala
    1115                 1120

<210>  47
<211>  496
<212>  PRT
<213>  Homo sapiens

<400>  47

Met Trp Gln Ile Val Phe Phe Thr Leu Ser Cys Asp Leu Val Leu Ala
1               5                   10                  15

Ala Ala Tyr Asn Asn Phe Arg Lys Ser Met Asp Ser Ile Gly Lys Lys
            20                  25                  30

Gln Tyr Gln Val Gln His Gly Ser Cys Ser Tyr Thr Phe Leu Leu Pro
        35                  40                  45

Glu Met Asp Asn Cys Arg Ser Ser Ser Ser Pro Tyr Val Ser Asn Ala
    50                  55                  60

Val Gln Arg Asp Ala Pro Leu Glu Tyr Asp Asp Ser Val Gln Arg Leu
65                  70                  75                  80

Gln Val Leu Glu Asn Ile Met Glu Asn Asn Thr Gln Trp Leu Met Lys
                85                  90                  95

Leu Glu Asn Tyr Ile Gln Asp Asn Met Lys Lys Glu Met Val Glu Ile
            100                 105                 110

Gln Gln Asn Ala Val Gln Asn Gln Thr Ala Val Met Ile Glu Ile Gly
        115                 120                 125

Thr Asn Leu Leu Asn Gln Thr Ala Glu Gln Thr Arg Lys Leu Thr Asp
    130                 135                 140

Val Glu Ala Gln Val Leu Asn Gln Thr Thr Arg Leu Glu Leu Gln Leu
145                 150                 155                 160

Leu Glu His Ser Leu Ser Thr Asn Lys Leu Glu Lys Gln Ile Leu Asp

                165                 170                 175

Gln Thr Ser Glu Ile Asn Lys Leu Gln Asp Lys Asn Ser Phe Leu Glu
            180                 185                 190

Lys Lys Val Leu Ala Met Glu Asp Lys His Ile Ile Gln Leu Gln Ser
        195                 200                 205

Ile Lys Glu Glu Lys Asp Gln Leu Gln Val Leu Val Ser Lys Gln Asn
    210                 215                 220

Ser Ile Ile Glu Glu Leu Glu Lys Lys Ile Val Thr Ala Thr Val Asn
225                 230                 235                 240

Asn Ser Val Leu Gln Lys Gln Gln His Asp Leu Met Glu Thr Val Asn
                245                 250                 255

Asn Leu Leu Thr Met Met Ser Thr Ser Asn Ser Ala Lys Asp Pro Thr
            260                 265                 270

Val Ala Lys Glu Glu Gln Ile Ser Phe Arg Asp Cys Ala Glu Val Phe
        275                 280                 285

Lys Ser Gly His Thr Thr Asn Gly Ile Tyr Thr Leu Thr Phe Pro Asn
    290                 295                 300

Ser Thr Glu Glu Ile Lys Ala Tyr Cys Asp Met Glu Ala Gly Gly Gly
305                 310                 315                 320

Gly Trp Thr Ile Ile Gln Arg Arg Glu Asp Gly Ser Val Asp Phe Gln
                325                 330                 335

Arg Thr Trp Lys Glu Tyr Lys Val Gly Phe Gly Asn Pro Ser Gly Glu
            340                 345                 350

Tyr Trp Leu Gly Asn Glu Phe Val Ser Gln Leu Thr Asn Gln Gln Arg
        355                 360                 365

Tyr Val Leu Lys Ile His Leu Lys Asp Trp Glu Gly Asn Glu Ala Tyr
    370                 375                 380

Ser Leu Tyr Glu His Phe Tyr Leu Ser Ser Glu Glu Leu Asn Tyr Arg
385                 390                 395                 400

Ile His Leu Lys Gly Leu Thr Gly Thr Ala Gly Lys Ile Ser Ser Ile
                405                 410                 415

Ser Gln Pro Gly Asn Asp Phe Ser Thr Lys Asp Gly Asp Asn Asp Lys
        420                 425                 430

Cys Ile Cys Lys Cys Ser Gln Met Leu Thr Gly Gly Trp Trp Phe Asp
        435                 440                 445

Ala Cys Gly Pro Ser Asn Leu Asn Gly Met Tyr Tyr Pro Gln Arg Gln
    450                 455                 460

Asn Thr Asn Lys Phe Asn Gly Ile Lys Trp Tyr Tyr Trp Lys Gly Ser
465                 470                 475                 480

Gly Tyr Ser Leu Lys Ala Thr Thr Met Met Ile Arg Pro Ala Asp Phe
                485                 490                 495

<210>  48
<211>  498
<212>  PRT
<213>  Homo sapiens

<400>  48

Met Thr Val Phe Leu Ser Phe Ala Phe Leu Ala Ala Ile Leu Thr His
1               5                   10                  15

Ile Gly Cys Ser Asn Gln Arg Arg Ser Pro Glu Asn Ser Gly Arg Arg
            20                  25                  30

Tyr Asn Arg Ile Gln His Gly Gln Cys Ala Tyr Thr Phe Ile Leu Pro
        35                  40                  45

Glu His Asp Gly Asn Cys Arg Glu Ser Thr Thr Asp Gln Tyr Asn Thr
    50                  55                  60

Asn Ala Leu Gln Arg Asp Ala Pro His Val Glu Pro Asp Phe Ser Ser
65                  70                  75                  80

Gln Lys Leu Gln His Leu Glu His Val Met Glu Asn Tyr Thr Gln Trp
                85                  90                  95

Leu Gln Lys Leu Glu Asn Tyr Ile Val Glu Asn Met Lys Ser Glu Met
            100                 105                 110

Ala Gln Ile Gln Gln Asn Ala Val Gln Asn His Thr Ala Thr Met Leu
        115                 120                 125

Glu Ile Gly Thr Ser Leu Leu Ser Gln Thr Ala Glu Gln Thr Arg Lys
    130                 135                 140

Leu Thr Asp Val Glu Thr Gln Val Leu Asn Gln Thr Ser Arg Leu Glu
145                 150                 155                 160

Ile Gln Leu Leu Glu Asn Ser Leu Ser Thr Tyr Lys Leu Glu Lys Gln
                165                 170                 175

Leu Leu Gln Gln Thr Asn Glu Ile Leu Lys Ile His Glu Lys Asn Ser
            180                 185                 190

Leu Leu Glu His Lys Ile Leu Glu Met Glu Gly Lys His Lys Glu Glu
        195                 200                 205

Leu Asp Thr Leu Lys Glu Glu Lys Glu Asn Leu Gln Gly Leu Val Thr
    210                 215                 220

Arg Gln Thr Tyr Ile Ile Gln Glu Leu Glu Lys Gln Leu Asn Arg Ala
225                 230                 235                 240

Thr Thr Asn Asn Ser Val Leu Gln Lys Gln Gln Leu Glu Leu Met Asp
                245                 250                 255

Thr Val His Asn Leu Val Asn Leu Cys Thr Lys Glu Gly Val Leu Leu
            260                 265                 270

Lys Gly Gly Lys Arg Glu Glu Glu Lys Pro Phe Arg Asp Cys Ala Asp
        275                 280                 285

Val Tyr Gln Ala Gly Phe Asn Lys Ser Gly Ile Tyr Thr Ile Tyr Ile
    290                 295                 300

Asn Asn Met Pro Glu Pro Lys Lys Val Phe Cys Asn Met Asp Val Asn
305                 310                 315                 320

Gly Gly Gly Trp Thr Val Ile Gln His Arg Glu Asp Gly Ser Leu Asp
                325                 330                 335

Phe Gln Arg Gly Trp Lys Glu Tyr Lys Met Gly Phe Gly Asn Pro Ser
            340                 345                 350

Gly Glu Tyr Trp Leu Gly Asn Glu Phe Ile Phe Ala Ile Thr Ser Gln
        355                 360                 365

Arg Gln Tyr Met Leu Arg Ile Glu Leu Met Asp Trp Glu Gly Asn Arg
    370                 375                 380

Ala Tyr Ser Gln Tyr Asp Arg Phe His Ile Gly Asn Glu Lys Gln Asn
385                 390                 395                 400

Tyr Arg Leu Tyr Leu Lys Gly His Thr Gly Thr Ala Gly Lys Gln Ser
                405                 410                 415

Ser Leu Ile Leu His Gly Ala Asp Phe Ser Thr Lys Asp Ala Asp Asn
            420                 425                 430

Asp Asn Cys Met Cys Lys Cys Ala Leu Met Leu Thr Gly Gly Trp Trp
        435                 440                 445

Phe Asp Ala Cys Gly Pro Ser Asn Leu Asn Gly Met Phe Tyr Thr Ala
    450                 455                 460

Gly Gln Asn His Gly Lys Leu Asn Gly Ile Lys Trp His Tyr Phe Lys
465                 470                 475                 480

Gly Pro Ser Tyr Ser Leu Arg Ser Thr Thr Met Met Ile Arg Pro Leu
                485                 490                 495

Asp Phe

<210>  49
<211>  330
<212>  PRT
<213>  Homo sapiens

<400>  49

<210>  50
<211>  326
<212>  PRT
<213>  Homo sapiens

<400>  50

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210> 51
<211> 377
<212> PRT
<213> Homo sapiens

<400> 51

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val
            180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210>  52

<211>  327
<212>  PRT
<213>  Homo sapiens

<400>  52

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Glu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys

225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  53
<211>  107
<212>  PRT
<213>  Homo sapiens

<400>  53

<210>  54

<211>  106
<212>  PRT
<213>  Homo sapiens

<400>  54

Gly Gln Pro Lys Ala Ala Pro Ser Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Ser Ser Pro
        35                  40                  45

Val Lys Ala Gly Val Glu Thr Thr Thr Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Arg Ser Tyr Ser Cys Gln Val Thr His Glu Gly Ser Thr Val
                85                  90                  95

Glu Lys Thr Val Ala Pro Thr Glu Cys Ser
            100                 105


<210>  55
<211>  448
<212>  PRT
<213>  Homo sapiens

<400>  55

Glu Val Gln Leu Val Gln Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Leu Asp Tyr Asp Leu Leu Thr Gly Tyr Gly Tyr Trp
        100                 105                 110

Gly Gln Gly Thr Leu Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Asn Phe Gly Thr Gln Thr Tyr Thr Cys Asn Val Asp
        195                 200                 205

His Lys Pro Ser Asn Thr Lys Val Asp Lys Thr Val Glu Arg Lys Cys
    210                 215                 220

Cys Val Glu Cys Pro Pro Cys Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Asn Ser Thr Phe Arg Val Val
    290                 295                 300

Ser Val Leu Thr Val Val His Gln Asp Trp Leu Asn Gly Lys Glu Tyr
305                 310                 315                 320

Lys Cys Lys Val Ser Asn Lys Gly Leu Pro Ala Pro Ile Glu Lys Thr
                325                 330                 335

Ile Ser Lys Thr Lys Gly Gln Pro Arg Glu Pro Gln Val Tyr Thr Leu

340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Met Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  56
<211>  59
<212>  PRT
<213>  Homo sapiens

<400>  56

Gly Gly Gly Gly Gly Ala Gln Gln Glu Glu Cys Glu Trp Asp Pro Trp
1               5                   10                  15

Thr Cys Glu His Met Gly Ser Gly Ser Ala Thr Gly Gly Ser Gly Ser
            20                  25                  30

Thr Ala Ser Ser Gly Ser Gly Ser Ala Thr His Gln Glu Glu Cys Glu
        35                  40                  45

Trp Asp Pro Trp Thr Cys Glu His Met Leu Glu
    50                  55

<210>  57
<211>  227
<212>  PRT
<213>  Homo sapiens

<400>  57

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

## Description

### FIELD OF THE INVENTION

The invention relates to methods for treating inflammatory conditions by administering one or more agents that inhibit angiopoietin-2 (ANG-2) function. Such agents can inhibit ANG-2 function by binding ANG-2 and/or TIE2 and inhibiting interaction between ANG-2 and TIE2 or by otherwise inhibiting the TIE2 response to ANG-2. In certain embodiments, the inflammatory condition involves inflammation in the central nervous system (CNS), including brain and spinal cord. Conditions include, but are not limited to, neurodegenerative diseases, such as multiple sclerosis. In one aspect, the ANG-2 inhibitor inhibits monocyte adhesion to vascular endothelial cells and the attendant development of monocytes into macrophages. Agents include, but are not limited to, antibodies that bind ANG-2 and/or TIE2 and fragments and variants thereof; non-antibody peptides that bind ANG-2, including, but not limited to, soluble TIE2 receptor and fragments and variants thereof, including the TIE2 extracellular domain; non-antibody peptides that bind TIE2, including ANG-2-based peptides; nucleotide-based inhibitors of ANG-2 expression; and small molecule inhibitors of ANG-2 or TIE2.

### BACKGROUND OF THE INVENTION

### Inflammation

Inflammation is the complex biological response of vascular tissues to harmful stimuli, such as pathogens, damaged cells, or irritants. It is a protective attempt by the organism to remove the injurious stimuli as well as initiate the healing process for the tissue. But inflammation that runs unchecked can lead to a host of diseases, including several in the CNS.

Inflammation can be classified as acute or chronic. Acute inflammation is the initial response of the body to harmful stimuli and is achieved by the increased movement of plasma and leukocytes from the blood into the injured tissues. A cascade of biochemical events propagates and matures the inflammatory response, involving the local vascular system, the immune system, and various cells within the injured tissue. Prolonged inflammation, known as chronic inflammation, leads to a progressive shift in the type of cells at the site of inflammation and is characterised by simultaneous destruction and healing of the tissue from the inflammatory process.

Acute inflammation is a short-term process that is characterized by the classic signs of inflammation - swelling, redness, pain, heat, and loss of function - due to the infiltration of the tissues by plasma and leukocytes. It occurs as long as the injurious stimulus is present and ceases once the stimulus has been removed, broken down, or walled off by scarring (fibrosis). The process of acute inflammation is initiated by the blood vessels local to the injured tissue, which alter to allow the exudation of plasma proteins and leukocytes into the surrounding tissue. The increased flow of fluid into the tissue causes the characteristic swelling associated with inflammation. The blood vessels undergo marked vascular changes, including vasodilation, increased permeability, and the slowing of blood flow, which are induced by the actions of various inflammatory mediators. Vasodilation occurs first at the arteriole level, progressing to the capillary level, and brings about a net increase in the amount of blood present, causing the redness and heat of inflammation. Increased permeability of the vessels results in the movement of plasma into the tissues, with resultant stasis due to the increase in the concentration of the cells within blood - a condition characterised by enlarged vessels packed with cells. Stasis allows leukocytes to marginate along the endothelium, a process critical to their recruitment into the tissues. Normal flowing blood prevents this, as the shearing force along the periphery of the vessels moves cells in the blood into the middle of the vessel. The changes thus permit the extravasation of leukocytes through the endothelium and basement membrane constituting the blood vessel. Once in the tissue, the cells migrate along a chemotactic gradient to reach the site of injury, where they can attempt to remove the stimulus and repair the tissue. Meanwhile, several biochemical cascade systems, consisting of chemicals known as plasma-derived inflammatory mediators, act in parallel to propagate and mature the inflammatory response. These include the complement system, coagulation system and fibrinolysis system. Finally, down-regulation of the inflammatory response concludes acute inflammation. Removal of the injurious stimuli halts the response of the inflammatory mechanisms, which require constant stimulation to propagate the process.

Leukocyte movement from the blood to the tissues through the blood vessels is known as extravasation, and can be divided up into a number of broad steps:

Leukocyte localisation and recruitment to the endothelium local to the site of inflammation - involving margination and adhesion to the endothelial cells: Recruitment of leukocytes is receptor-mediated. The products of inflammation, such as histamine, promote the immediate expression of P-selectin on endothelial cell surfaces. This receptor binds weakly to carbohydrate ligands on leukocyte surfaces and causes them to "roll" along the endothelial surface as bonds are made and broken. Cytokines from injured cells induce the expression of E-selectin on endothelial cells, which functions similarly to P-selectin. Cytokines also induce the expression of integrin ligands on endothelial cells, which further slow leukocytes down. These weakly bound leukocytes are free to detach if not activated by chemokines produced in injured tissue. Activation increases the affinity of bound integrin receptors for ligands on the endothelial cell surface, firmly binding the leukocytes to the endothelium.

Migration across the endothelium, known as transmigration, via the process of diapedesis: Chemokine gradients stimulate the adhered leukocytes to move between endothelial cells and pass the basement membrane into the tissues.

Movement of leukocytes within the tissue via chemotaxis: Leukocytes reaching the tissue interstitium bind to extracellular matrix proteins via expressed integrins and CD44 to prevent their loss from the site. Chemoattractants cause the leukocytes to move along a chemotactic gradient towards the source of inflammation.

Chronic inflammation is a pathological condition characterised by concurrent active inflammation, tissue destruction, and attempts at repair. Chronic inflammation is not characterised by the classic signs of acute inflammation listed above. Instead, chronically inflamed tissue is characterised by the infiltration of mononuclear immune cells (monocytes, macrophages, lymphocytes, and plasma cells), tissue destruction, and attempts at healing, which include angiogenesis and fibrosis. Endogenous causes include persistent acute inflammation. Exogenous causes are varied and include bacterial infection, especially by Mycobacterium tuberculosis, prolonged exposure to chemical agents such as silica, tobacco smoke, or autoimmune reactions such as rheumatoid arthritis.

In acute inflammation, removal of the stimulus halts the recruitment of monocytes (which become macrophages under appropriate activation) into the inflamed tissue, and existing macrophages exit the tissue via lymphatics. However in chronically inflamed tissue the stimulus is persistent, and therefore recruitment of monocytes is maintained, existing macrophages are tethered in place, and proliferation of macrophages is stimulated (especially in atheromatous plaques).

The cellular component of inflammation involves leukocytes, which normally reside in blood and must move into the inflamed tissue via extravasation to aid in inflammation. Some act as phagocytes, ingesting bacteria, viruses, and cellular debris. Others release enzymatic granules which damage pathogenic invaders. Leukocytes also release inflammatory mediators which develop and maintain the inflammatory response. Generally speaking, acute inflammation is mediated by granulocytes, while chronic inflammation is mediated by mononuclear cells such as monocytes and lymphocytes.

### Monocyte

A monocyte is one type of leukocyte, part of the human body's immune system. Monocytes have two main functions in the immune system: (1) replenish resident macrophages and dendritic cells under normal states, and (2) in response to inflammation signals, monocytes can move quickly (approx. 8-12 hours) to sites of infection in the tissues and differentiate into macrophages and dendritic cells to elicit an immune response. Monocytes are usually identified in stained smears by their large bilobate nucleus.

Monocytes are produced by the bone marrow from hematopoietic stem cell precursors called monoblasts. Monocytes circulate in the bloodstream for about one to three days and then typically move into tissues throughout the body. In the tissues, monocytes mature into different types of macrophages at different anatomical locations. Macrophages are responsible for protecting tissues from foreign substances but are also suspected to be the predominant cells involved in triggering atherosclerosis.

Monocytes are responsible for phagocytosis (ingestion) of foreign substances in the body. Monocytes can perform phagocytosis using intermediary (opsonizing) proteins such as antibodies or complement that coat the pathogen, as well as by binding to the microbe directly via pattern-recognition receptors that recognize pathogens. Monocytes are also capable of killing infected host cells via antibody, termed antibody-mediated cellular cytotoxicity.

Examples of processes that can increase monocyte count include: chronic inflammation, stress response, hyperadrenocorticism, immune-mediated disease, pyogranulomatous disease, necrosis, and red cell regeneration.

### TIE2/TEK Tyrosine Kinase

The receptor tyrosine kinases (RTKs) are a large and evolutionarily conserved family of proteins involved in the transduction of extracellular signals to the cytoplasm. Among the RTKs believed to be involved in vascular morphogenesis and maintenance are the vascular endothelial growth factor (VEGF) receptors and TIE2 (also known as Tek and ork) (see Hanahan, Science 277:48, 1997). TIE2 is an RTK that is predominantly expressed in vascular endothelium. This receptor possesses an extracellular domain containing two immunoglobulin-like loops separated by three epidermal growth factor-like repeats that are connected to 3 fibronectin type III-like repeats. Disruption of the TIE2 gene by homologous recombination in ES cells results in embryonic lethality in homozygous mutant mouse embryos due to deficiency of the endothelium. The molecular cloning of human TIE2 has been described by Ziegler, U.S. Pat. No. 5,447,860. Four TIE2 ligands, angiopoietin-1, ANG-2, angiopoietin-3, and angiopoietin-4 (ANG-1, ANG-2, ANG-3, and ANG-4), have been described (Davis et al., Cell 87:1161 (1996); Maisonpierre et al., Science 277:55 (1997); Valenzuela et al., Proc Natl Acad Sci USA 96:1904 (1999)). These ligands have distinct expression patterns and activities with respect to TIE2. "Tie ligand homologs" designated NL1, NL5, NL8, and NL4 are described in U.S. Pat. No. 6,057,435. ANG-1 causes receptor activation and autophosphorylation. ANG-2 blocks autophosphorylation and causes receptor inactivation.

### Angiopoietin-2

ANG-2 is a naturally occurring antagonist of ANG-1 that competes for binding to the TIE2 receptor and blocks ANG-1-induced TIE2 autophosphorylation during vasculogenesis (Kim et al., J Biol Chem, 275:18550-18556 (2000). Maisonpierre, (Science, 277:55-60 (1997)) identified ANG-2, and showed that it is a naturally occurring antagonist for both ANG-1 and TIE2. The investigators found that the predicted ANG-2 protein is 496 amino acids long and has a secretion signal peptide. Human and mouse ANG-2 are 85% identical in amino acid sequence and approximately 60% identical to their ANG-1 homologs. Transgenic overexpression of ANG-2 in mice disrupted blood vessel formation in the mouse embryo. In adult mice and humans, they found that ANG-2 is expressed only at the sites of vascular remodeling.

Ward (Cytogenet Cell Genet, 94:147-154 (2001)) determined that the ANG-2 gene contains 9 exons and spans 32.3 kb. Exons 1 to 5 encode the N terminus, the coiled-coil domain, and part of the hinge region, and exons 5 to 9 encode the remainder of the hinge region, the fibrinogen-like domain, and the C terminus.

### SUMMARY OF THE INVENTION

The invention is broadly directed to a method for treating an inflammatory condition by inhibiting ANG-2. In one embodiment, the invention is directed to treating an inflammatory condition that is not associated with detrimental angiogenesis.

The invention is also broadly directed to a method for reducing (preventing or decreasing) monocyte adhesion to endothelial cells by inhibiting ANG-2.

The invention is also broadly directed to a method for reducing the development of perivascular macrophages from circulating monocytes by inhibiting ANG-2.

The invention is also broadly directed to a method for reducing the number of perivascular macrophages by inhibiting ANG-2.

The invention is also broadly directed to a method of reducing monocyte migration across the endothelium by inhibiting ANG-2.

The invention is also broadly directed to a method for reducing monocyte migration into the perivasculature by inhibiting ANG-2.

The invention is also broadly directed to a method for reducing the interaction of ANG-2 with monocytes.

The invention is more specifically directed to each of the above methods in the CNS. This includes brain and spinal cord. However, the methods particularly relate to the brain.

Accordingly, all of these methods relate to endothelial cells that constitute the blood-brain barrier.

Thus, in one embodiment the invention is directed to a method for preventing monocytes from crossing the blood-brain barrier.

The invention is also directed to a method of blocking infiltration of monocytes into nervous tissue (e.g. CNS) during inflammatory conditions.

The invention is also directed to a method of reducing the adhesion of monocytes to the cerebral vasculature.

The invention is also directed to a method of reducing the activation of cerebral macrophages.

In one embodiment, the condition that is treated is inflammation of the CNS. This includes conditions involving the brain and/or spinal cord. These conditions include, but are not limited to, neurodegenerative diseases, such as Alzheimer's Disease, Parkinson's Disease, multiple sclerosis, amylotropic lateral sclerosis (Lou Gehrig's Disease), and Creutzfeldt-Jakob Disease (CJD); acute CNS injuries, such as spinal cord traumatic injury, brain traumatic injury, stroke, cerebral ischemia, and epilepsy; psychiatric disorders, such as depression, bi-polar disorder, and schizophrenia. In one embodiment, the condition that is treated is not glioma. A preferred embodiment is treatment of multiple sclerosis.

The invention is directed to any inflammatory condition in which monocyte adhesion to endothelium is undesirable and causes or may cause (should it occur) clinically detrimental symptoms in a subj ect.

In one embodiment, the monocytes are CD68⁺GR1⁻. However, they may be GR1⁺.

ANG-2 is inhibited by administering a therapeutically effective amount of one or more ANG-2 inhibitors, such as those described herein, to a subject in need of treatment. In preferred embodiments, the subject is a mammal and, particularly, human.

Mechanisms by which inhibition can be achieved include, but are not limited to, inhibition of binding of ANG-2 to TIE2, inhibition of TIE2 inactivation and allowing autophosphorylation, and increased clearance of ANG-2 from a patient's body, thus reducing the effective concentration of ANG-2. It is understood that, whatever the mechanism, the end result of inhibition is preventing the inactivation of TIE2 that is normally achieved by ANG-2.

ANG-2 inhibitors include, but are not limited to, agents that specifically bind ANG-2. These include antibodies or antibody-based agents (i.e., where antigen-binding is by specific recognition of the antigen by variable region sequences or derivatives of such sequences). Antibodies include natural and recombinant antibodies, variants, fragments and derivatives. Fragments include any that comprise the antigen specificity regions, including an Fab fragment, an Fab' fragment, an F(ab')₂ fragment, an Fv fragment, a diabody, or a single chain antibody, chimeric antibodies, including humanized antibodies and multi-specific antibodies. Antibodies include fully human antibodies. Antibodies include monoclonal or polyclonal antibodies. A human antibody includes the IgG1-, IgG2- IgG3- or IgG4-type.

Inhibitors that specifically bind ANG-2 also include non-variable region-based peptides. Such peptides may be based on TIE2 sequences, including soluble TIE2 or variants or fragments of TIE2. In one embodiment, the fragment comprises the TIE2 extracellular domain or variants or fragments thereof that specifically bind ANG-2.

In another embodiment, the inhibitors comprise non-variable region peptides, such as synthetic random peptides or random naturally-occurring peptides that bind to ANG-2 or variants or fragments of these peptides.

In another embodiment, inhibition is effected through agents that specifically bind TIE2. These include TIE2 antibodies or variants or fragments thereof as described above for agents that bind ANG-2.

In another embodiment, inhibitors that bind TIE2 may be based on the ANG-2 sequence. This includes ANG-2 variants and fragments.

In another embodiment, the inhibitors comprise non-variable region-based peptides, such as synthetic random peptides or random naturally-occurring peptides, that specifically bind TIE2, as well as variants and fragments of these peptides.

In specific embodiments, the antibody that recognizes ANG-2 comprises a heavy chain variable region selected from the group consisting of SEQ ID NO: 1; SEQ ID NO: 2; SEQ ID NO: 3; SEQ ID NO: 4; SEQ ID NO: 5; SEQ ID NO: 6; SEQ ID NO: 7 and a light chain variable region selected from the group consisting of SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16; SEQ ID NO: 17; as well as antigen binding fragments thereof. These are shown in Tables 2 and 3 herein.

In another embodiment, the inhibitor comprises a specific binding agent comprising heavy chain complementarity determining region 1 (CDR 1): SEQ ID NO: 18.

In another embodiment, the inhibitor comprises a specific binding agent comprising heavy chain complementarity determining region 2 (CDR 2) of any of: SEQ ID NO: 19; SEQ ID NO: 20; SEQ ID NO: 21.

In another embodiment, the inhibitor comprises a specific binding agent comprising heavy chain complementarity determining region 3 (CDR 3) of any of: SEQ ID NO: 22; SEQ ID NO: 23; SEQ ID NO: 24; SEQ ID NO: 25; SEQ ID NO: 26; SEQ ID NO: 27.

In another embodiment, the inhibitor comprises a specific binding agent comprising light chain complementarity determining region 1 (CDR 1) of any of: SEQ ID NO: 28; SEQ ID NO: 29; SEQ ID NO: 30; SEQ ID NO: 31; SEQ ID NO: 32; SEQ ID NO: 33; SEQ ID NO: 34.

In another embodiment, the inhibitor comprises a specific binding agent comprising light chain complementarity determining region 2 (CDR 2) of any of: SEQ ID NO: 35; SEQ ID NO: 36; SEQ ID NO: 37.

In another embodiment, the inhibitor comprises a specific binding agent comprising light chain complementarity determining region 3 (CDR 3) of any of: SEQ ID NO: 38; SEQ ID NO: 39; SEQ ID NO: 40.

The inhibitor can combine one or more of any of the CDRs above. In some embodiments, the inhibitor comprises three CDRs, e.g., a CDR1, CDR2, and CDR3.

Specific ANG-2 inhibitor antibodies also include an antibody designated 536 (Tables 2 and 4) comprising a heavy chain and a light chain, where the heavy chain contains the variable region EVQLVQSGGGVVQPGRSLRLSCAASGFTFSSYGMHWVRQAPG KGLEWVSYISSSGSTIYYADSVKGRFTISRDNAKNSLYLQMNSLRAEDTAVYYCARD LLDYDILTGYGYWGQGTLVTVSS (SEQ ID NO: 41); and antigen binding fragments thereof; and the light chain comprises the variable region DIVMTQSPLSLPVTPGEPA SISCRSSQSLLHSNGYNFLDWYLQKPGQSPQLLIYLGSNRASGVPDRFSGSGSGTDFT LKISRVEAEDVGVYYCMQGTHWPPTFGQGTKLEIK (SEQ ID NO: 42) or DIVMTQSP LSLPVTPGEPASISCRSSQSLLHSNGYNYLDWYLQKPGQSPQLLIYLGSNRASGVPDR FSGSGSGTDFTLKISRVEAEDVGVYYCMQGLQTPPTFGQGTKLEIK (SEQ ID NO: 43); and antigen binding fragments thereof.

Specific ANG-2 inhibitor antibodies also include an antibody designated H4L4 (Tables 1-4) comprising a heavy chain and a light chain, wherein the heavy chain comprises the heavy chain variable region H4 (SEQ ID NO: 3); and antigen binding fragments thereof; and the light chain comprises the light chain variable region L4 (SEQ ID NO: 10); and antigen binding fragments thereof. The H4 sequence is EVQLVQSGGGVVQPGRSLRLSC ASGFTFSSYGMHWVRQAPGKGLEWVSYISSSGSTIYYADSVKGRFTISRDNAKNSLY QMNSLRAEDTAVYYCARDLLDYDLLTGYGYWGQGTLVTVSS. The L4 sequence is DIVMTQSPLSLPVTPGEPASISCRSSQSLLHSHYNYLDWYLQKPGQSPQLLIYLGSNRS GVPDRFSGSGSGTDFTLKISRVEAEDVGVYYCMQGTHWPPTFGQGTKLEIK.

Inhibition of ANG-1 may also be beneficial. Therefore, in another embodiment, the inhibitor specifically recognizes ANG-1, as well as ANG-2. The degree of inhibition of ANG-1 can vary.

In one embodiment, the specifically binding peptides described above are attached to vehicles. Vehicles include both peptide and non-peptide-based vehicles. Peptide-based vehicles include fusion to Fc domains, thereby providing peptibodies. In a specific embodiment, the peptibody is based on TIE2 and may be designated a "receptorbody" or based on ANG-2 and designated a "ligandbody".

In one specific embodiment, the specific binding agent comprises the sequence TNFMPMDDLEQRLYEEQFILQQG (SEQ ID NO: 44). This can be combined with an F_{C} fragment to form a peptibody designated L1-7.

In one specific embodiment, the specific binding agent comprises the sequence QKFQPLDELEQTLYEQFMLQQA (SEQ ID NO: 45). Oliner et al., Cancer Cell, 6:507-516 (2004). This can be combined with an F_{C} fragment to form a peptibody designated L1-10.

In further embodiments, the ANG-2 inhibitors comprise small organic molecules, mimetics, avimers, and aptamers.

ANG-2 inhibitors also include agents that reduce expression (i.e., transcription, translation, translocation, or secretion) of ANG-2. These include, but are not limited to, antisense polynucleotides, polynucleotides useful for RNA interference, ribozymes, small organic molecules, and nucleic acid aptamers.

Accordingly, the invention is directed to methods of treatment with a pharmaceutical composition comprising one or more ANG-2 inhibitors, such as those described herein, and a pharmaceutically acceptable carrier.

In one embodiment, the treatment is solely with an ANG-2 inhibitor. In other embodiments, the inhibitor is administered in combination with one or more additional therapeutic agents. In one specific embodiment, this combination does not include administering an ANG-2 inhibitor in combination with nerve growth factor.

The invention specifically relates to methods for treating inflammation using, in combination, an ANG-2 inhibitor and another anti-inflammatory agent. In one embodiment, the anti-inflammatory agent comprises a disease-modifying anti-rheumatic drug (DMARD), slow acting anti-rheumatic drug (SAARD), or non-steroidal anti-inflammatory drug (NSAID). In another embodiment, the anti-inflammatory agent comprises methotrexate, a tumor necrosis factor (TNF) inhibitor, an IL-1 inhibitor, a TACE inhibitor, a COX-2 inhibitor, and a P-38 inhibitor. In still another embodiment, the TNF inhibitor comprises at least one of etanercept, adalimumab, pegsunercept sTNF-R1, onercept, and infliximab. In yet another embodiment, the IL-1 inhibitor may be at least one of anakinra, IL-1 TRAP, IL-1 antibody, and soluble IL-1 receptor. In a specific embodiment, the ANG-2 inhibitor is administered in combination with TNF.

These and other aspects will be described in greater detail herein. Each of the aspects provided can encompass various embodiments provided herein. It is therefore anticipated that each of the embodiments involving one element or combinations of elements can be included in each aspect described. Other features, objects, and advantages of the disclosed invention are apparent in the detailed description that follows.

### BRIEF DESCRIPTION OF THE DRAWINGS

Figure 1. Single-dose prophylactic treatment with anti-ANG-2 monoclonal antibody H4L4 ameliorates disease in SJL/PLP₁₃₉₋₁₅₁ EAE compared to treatment with isotype control.

Figure 2. Single-dose prophylactic treatment with anti-ANG-2 peptibody L1-7 ameliorates disease in SJL/PLP 139-151 EAE compared to treatment with human Fc control.

Figure 3A is the amino acid sequence of human TIE2 (SEQ ID NO: 46) (Swiss Prot Acc. No. Q02763).

Figure 3B shows the putative extracellular, transmembrane, and cytoplasmic domains in TIE 2.

Figure 3C is the amino acid sequence of human ANG-2 (SEQ ID NO: 47) (GenBank Acc. No. NP001138).

Figure 3D is the amino acid sequence of human ANG-1 (SEQ ID NO: 48) (GenBank Acc. No. NP001137).

### DETAILED DESCRIPTION OF THE INVENTION

It should be understood that this invention is not limited to the particular methodology, protocols, and reagents, etc., described herein and, as such, may vary. The terminology used herein is for the purpose of describing particular embodiments only, and is not intended to limit the scope of the disclosed invention, which is defined solely by the claims.

The section headings are used herein for organizational purposes only and are not to be construed as in any way limiting the subject matter described.

The methods and techniques of the present application are generally performed according to conventional methods well-known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane, Antibodies: A Laboratory Manual, Cold Spring Harbor Laboratory Press, Cold Spring Harbor, N.Y. (1990).

### Definitions

"A" or "an" means herein one or more than one; at least one. Where the plural form is used herein, it generally includes the singular.

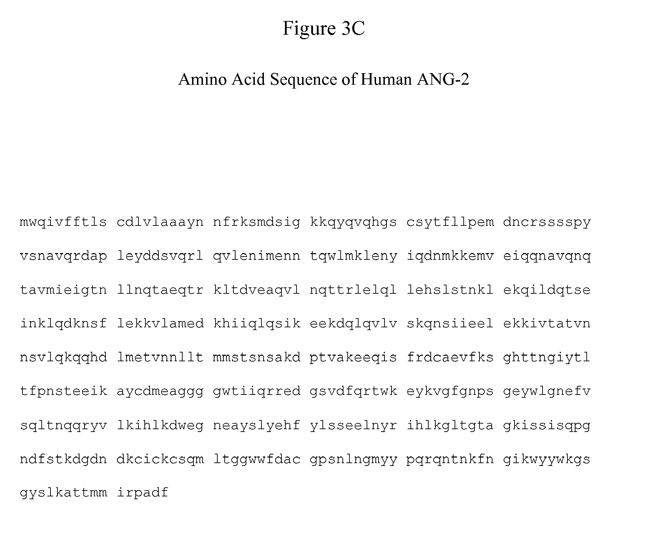

"ANG-2" is a designation for angiopoietin-2. The term "ANG-2" refers to the polypeptide set forth in Figure 6 of U.S. Patent No. 6,166,185 and in Figure 3C herein (SEQ ID NO: 47).

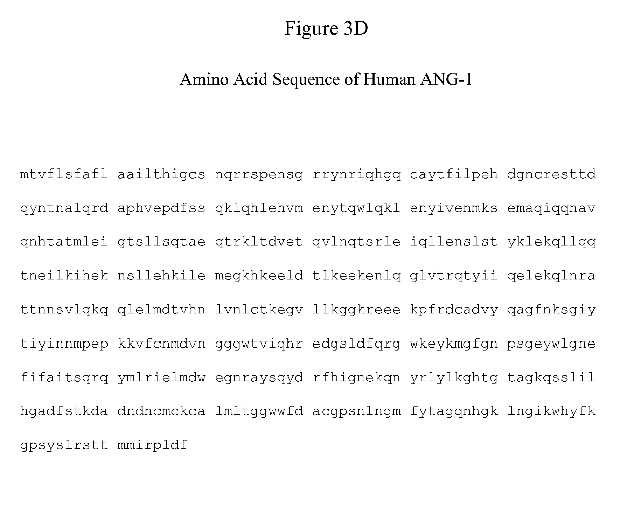

"ANG-1" is a designation for angiopoietin-1. The term "ANG-1" refers to the polypeptide set forth in Figure 3D herein (SEQ ID NO: 48).

The terms ANG-2 and TIE2 also refer to fragments and variants including allelic variants, orthologs, splice variants, substitution, deletion and insertion variants. They also refer to derivatives, including proteins, multimers, chemically-modified forms, and randomized sequence forms. The ANG-2 polypeptide may or may not include additional terminal residues, e.g., leader sequences, targeting sequences, amino terminal methionine, amino terminal methionine and lysine residues, and/or tag or fusion proteins sequences, depending on the manner in which it is prepared.

An "ANG-2 inhibitor" as used herein encompasses compounds that interfere with one or more ANG-2 functions. Normally, ANG-2 acts by binding to TIE2 and preventing autophosphorylation (i.e., inactivates the receptor). Therefore, in general, ANG-2 inhibitors reduce TIE2 inactivation/allow auto-phosphorylation. This includes ANG-2 binding to the TIE2 receptor, ANG-2 gene expression, or ANG-2 interaction with other molecules by which ANG-2 function is facilitated. Thus, ANG-2 inhibition can occur by several different means. In one embodiment, the inhibitor acts by blocking the binding of ANG-2 to the TIE2 receptor. The inhibitor may bind to ANG-2 and thereby prevent its binding to TIE2. This inhibitor may be a competitive inhibitor of ANG-2 and may effect inhibition by binding to the TIE2 receptor at the ANG-2 binding site. In another embodiment, an inhibitor (e.g., a modified ANG-2) may bind to the receptor, but fail to effect signal transduction (i.e., fail to prevent or reduce autophosphorylation of the receptor). In another embodiment, the inhibitor may compete with the receptor itself for binding to ANG-2, for example, soluble receptor and receptor derivatives. ANG-2 inhibitors may act by binding to factors that bind to or are bound by ANG-2. It is understood that the ANG-2 inhibitors can operate by one or more of these mechanisms as well.

ANG-2 inhibitors can be, to name just a few examples, small molecules, peptides, polypeptides, proteins, including more specifically antibodies, including anti-Ang2 antibodies, intrabodies, maxibodies, minibodies, diabodies, Fc fusion proteins such as peptibodies, receptibodies, soluble TIE2 receptor proteins and fragments, and a variety of others.

"Co-administer"" means to administer in conjunction with one another, together, coordinately, including simultaneous or sequential administration of two or more agents.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of and similar phrases using words of the root "comprise" are used herein as synonyms of "comprising" and have the same meaning.

"Comprised of" is a synonym of comprising (see above).

"Effective amount" generally means an amount which provides the desired local or systemic effect. For example, an effective amount is an amount sufficient to effectuate a beneficial or desired clinical result. The effective amounts can be provided all at once in a single administration or in fractional amounts that provide the effective amount in several administrations. The precise determination of what would be considered an effective amount may be based on factors individual to each subject, including their size, age, injury, and/or disease or injury being treated, and amount of time since the injury occurred or the disease began. One skilled in the art will be able to determine the effective amount for a given subject based on these considerations which are routine in the art. As used herein, "effective dose" means the same as "effective amount."

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

Use of the term "includes" is not intended to be limiting. For example, stating that the antibody inhibitor "includes" fragments and variants does not mean that other forms of the antibody inhibitor are excluded.

A "peptibody," in general, refers to molecules comprising at least part or all of an immunoglobulin Fc domain and at least one peptide. Such peptibodies may be multimers or dimers or fragments thereof, and they may be derivatized. Peptibodies are known in the art and are described in greater detail in WO 99/25044 and WO 00/24782, which are incorporated herein by reference for teaching peptibody technology.

"Pharmaceutically acceptable derivative" is any pharmaceutically acceptable derivative of a compound of the invention, such as a salt, an ester, a metabolite, or a residue of a compound of this invention.

The term "reduce" as used herein means to prevent as well as decrease.

"Specifically binds ANG-2" refers to the ability of the inhibitor to recognize and bind an ANG-2 polypeptide, such that its affinity (as determined by, e.g., Affinity ELISA or BIAcore assays as described herein) or its neutralization capability (as determined by e.g., Neutralization ELISA assays described herein, or similar assays) is at least 10 times as great, but optionally 50 times as great, 100, 250 or 500 times as great, or even at least 1000 times as great as the affinity or neutralization capability of the same for any other angiopoietin (e.g., ANG-1, ANG-3, ANG-4) or other peptide or polypeptide. Specific binding agents include proteins, peptides, nucleic acids, carbohydrates, lipids, or small molecular weight compounds which bind preferentially to ANG-2.

An ANG-2 inhibitor is said to "specifically bind" its target when the dissociation constant (K_{d}) is ≤10⁻⁸ M. The ANG-2 inhibitor specifically binds its target with "high affinity" when the K_{d} is ≤5x 10⁻⁹ M, and with "very high affinity" when the K_{d} is ≤5x10⁻¹⁰ M. In one embodiment, the ANG-2 inhibitor has a K_{d} of ≤10⁻⁹ M and an off-rate of about 1x 10⁻⁴/sec. In one embodiment, the off-rate is <1x10⁻⁵. In other embodiments, the ANG-2 inhibitors will bind to ANG-2 or TIE2 gene, gene transcript, and/or protein, full-length or fragment thereof, with a K_{d} of between about 10⁻⁸ M and 10⁻¹⁰ M, and in yet another embodiment it will bind with a K_{d} ≤2x10⁻¹⁰.

"Subject" means a vertebrate, such as a mammal, such as a human. Mammals include, but are not limited to, humans, dogs, cats, horses, cows, and pigs.

The term "therapeutically effective amount" refers to the amount of an ANG-2 inhibitor determined to produce any therapeutic response in a mammal. For example, effective anti-inflammatory therapeutic agents may prolong the survivability of the patient, and/or inhibit overt clinical symptoms. Treatments that are therapeutically effective within the meaning of the term as used herein, include treatments that improve a subject's quality of life even if they do not improve the disease outcome per se. Such therapeutically effective amounts are readily ascertained by one of ordinary skill in the art. Thus, to "treat" means to deliver such an amount. Thus, treating can prevent or ameliorate any pathological symptoms of inflammation.

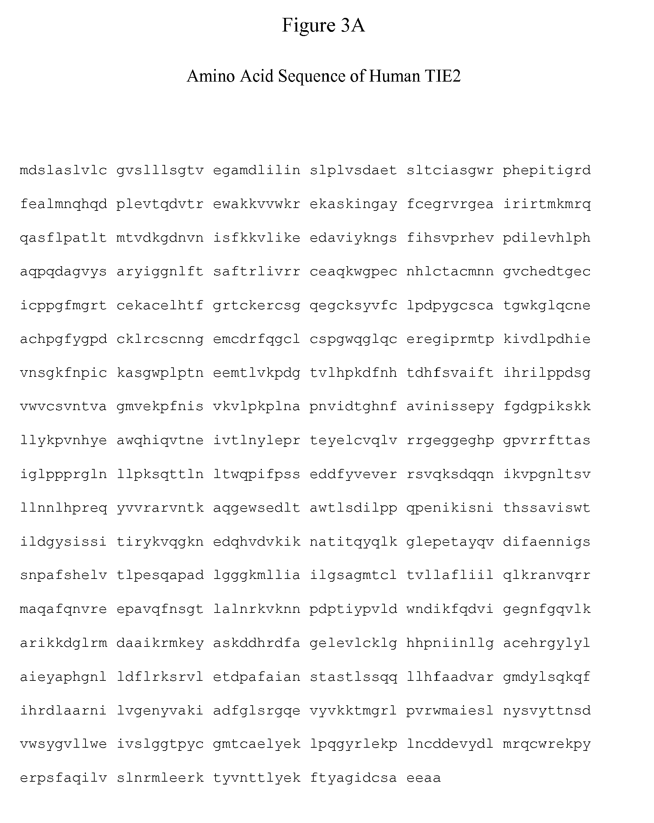

The term "TIE2" refers to the receptor to which ANG-2 normally binds. The native TIE2 sequence is set forth in Figure 3A (SEQ ID NO: 46).

"Treat," "treating," or "treatment" are used broadly in relation to the invention and each such term encompasses, among others, preventing, ameliorating, inhibiting, or curing a deficiency, dysfunction, disease, or other deleterious process, including those that interfere with and/or result from a therapy.

"Variants" have one or more amino acid deletions, insertions or substitutions. Amino acid substitutions (whether conservative or non-conservative) of the subject antibodies or peptides can be implemented by those skilled in the art by applying routine techniques. One skilled in the art may generate test variants containing a single amino acid substitution at each desired amino acid residue. The variants can then be screened using activity assays known to those skilled in the art. The importance of the hydropathic profile in conferring interactive biological function on a protein is understood in the art (see, e.g., Kyte et al., J Mol Biol, 157:105-131 (1982)). Examples of art-recognized polypeptide secondary and tertiary structures are described in Proteins, Structures and Molecular Principles (Creighton, Ed.), 1984, W.H. New York: Freeman and Company; Introduction to Protein Structure (Branden and Tooze, ed.), 1991, New York: Garland Publishing; and Thornton et al., Nature, 354:105 (1991).

Variants may: (1) reduce susceptibility to proteolysis, (2) reduce susceptibility to oxidation, (3) increase binding affinity for ANG-2, and/or (4) confer or modify other physicochemical or functional properties on such polypeptides.

### ANG-2 Inhibitors

### I. Antibodies

Antibody-based inhibitors that are immunoreactive with epitopes of ANG-2 and/or TIE2 may be used. Antibody-based inhibitors are those that recognize the antigen (i.e., ANG-2 or TIE2) by means of antibody specificity sequences (variable region sequences or fragments or variants thereof). The term "antibody" includes, in addition to antibodies comprising two full-length heavy chains and two full-length light chains, variants, fragments, and derivatives thereof. A "variant" comprises an amino acid sequence wherein one or more amino acid residues are inserted into, deleted from and/or substituted into the amino acid sequence relative to another polypeptide sequence. A "derivative" has been chemically modified in some manner distinct from insertion, deletion, or substitution variants, e.g., *via* conjugation to another chemical moiety. In certain embodiments, the antibodies include, but are not limited to, polyclonal antibodies, monoclonal antibodies, bispecific antibodies, minibodies, domain antibodies, synthetic antibodies (sometimes referred to as "antibody mimetics"), chimeric antibodies, humanized antibodies, human antibodies, and antibody fusions (sometimes referred to as "antibody conjugates"). Useful fragments include immunologically functional fragments of antibodies, including specific binding domains (e.g., a CDR region or a variable domain of a heavy or light chain).

For the production of antibodies, various host animals can be immunized by injection with one or more of the following: an ANG-2 polypeptide, a fragment of an ANG-2 polypeptide, a functional equivalent of an ANG-2 polypeptide, a variant of ANG-2; a TIE2 polypeptide, a fragment of a TIE2 polypeptide, a functional equivalent of a TIE2 polypeptide, or a variant of a TIE2 polypeptide. Fragments can include one or more epitopes alone or in combination with a peptide vehicle.

### A. Polyclonal Antibodies

Polyclonal antibodies comprise a heterogeneous mixture of antibodies that recognize and bind to different epitopes on the same antigen. Polyconal antibodies can be prepared by immunizing an animal (or human) using standard procedures and isolating the antibodies according to well-known methods. Polyclonal antibodies may be obtained from crude serum preparations or may be purified using, for example, antigen affinity chromatography, or Protein A/Protein G affinity chromatography. See, for example, Antibodies, A Laboratory Manual, Harlow et al.; Cold Spring Harbor Laboratory (1988).

### B. Monoclonal Antibodies

Monoclonal antibodies can be produced using any technique known in the art, e.g., by immortalizing spleen cells harvested from a transgenic animal after completion of the immunization schedule. Such monoclonal antibodies can be of any immunoglobulin class including IgG, IgM, IgE, IgA, IgD and any subclass thereof. See, for example, Monoclonal Antibodies, Hybridomas: A New Dimension in Biological Analyses, Kennet et al. (eds.), Plenum Press, New York (1980); and Antibodies: A Laboratory Manual, Harlow and Land (eds.), Cold Spring Harbor Laboratory Press, Cold Spring Harbor, N.Y., (1988); Kohler and Milstein, (U.S. Pat. No. 4,376,110); the human B-cell hybridoma technique (Kozbor et al., J Immunol, 133:3001-3005 (1984); Cole et al., Proc Natl Acad Sci USA, 80:2026-2030 (1983)); and the EBV-hybridoma technique (Cole et al., Monoclonal Antibodies And Cancer Therapy, Alan R. Liss, Inc., pp. 77-96 (1985)).

### C. Chimeric Antibodies

Generally, the goal of making a chimeric antibody is to create a chimera in which the number of amino acids from the intended patient species is maximized to avoid adverse immunological reactions. For methods relating to chimeric antibodies, see, for example, U.S. 4,816,567; and Morrison et al., Proc Natl Acad Sci USA, 81:6851-6855 (1985).

### 1. Peptides Based on Complementarity Determining Regions (CDRs)

In one embodiment, the ANG-2 inhibitors comprise one or more CDRs that are derived from or based on naturally-occurring variable regions. A variable region comprises at least three heavy or light chain CDRs. Thus, ANG-2 inhibitors may comprise (a) a polypeptide structure and (b) one or more CDRs that are inserted into and/or joined to the polypeptide structure. The polypeptide structure can take a variety of different forms. For example, it can comprise the framework of a naturally-occurring antibody, or fragment or variant thereof, or may be completely synthetic in nature.

The antibody can comprise one or more CDRs from a particular species or belonging to a particular antibody class or subclass, while the remainder of the antibody chain(s) is/are identical with or homologous to a corresponding sequence in antibodies derived from another species or belonging to another antibody class or subclass. CDRs and framework regions (FR) of a given antibody may be identified using the numbering system defined in Kabat et al., Sequences of Proteins of Immunological Interest, 5th Ed., US Dept. of Health and Human Services, PHS, NIH, NIH Publication No. 91-3242, 1991, or Chothia & Lesk, J Mol Biol, 196:901-917 (1987); Chothia et al., Nature, 342:878-883 (1989). CDR grafting is described, for example, in U. S. Patent Nos. 6,180,370; 5,693,762; 5,693,761; 5,585,089; and 5,530,101.

The inserts may represent, for example, a completely degenerate or biased array. One then can select phage-bearing inserts that bind to the desired antigen. This process can be repeated through several cycles of reselection of phage that bind to the desired antigen. Repeated rounds lead to enrichment of phage bearing particular sequences. DNA sequence analysis can be conducted to identify the sequences of the expressed peptides. The minimal linear portion of the sequence that binds to the desired antigen can be determined. One can repeat the procedure using a biased library containing inserts containing part or all of the minimal linear portion plus one or more additional degenerate upstream or downstream residues.

Alternatively, grafted variable regions from a heavy or light chain may be combined with a constant region that is different from the constant region of that particular heavy or light chain. In other embodiments, the grafted variable regions are part of a single chain Fv antibody.

### 2. Humanized Antibodies

For use in humans, the variable region or selected CDRs from a rodent antibody often are grafted into a human antibody, replacing the naturally-occurring variable regions or CDRs of the human antibody. Accordingly, one useful type of chimeric antibody is a "humanized" antibody. Humanization can be performed, for example, using various methods by substituting at least a portion of a rodent variable region for the corresponding regions of a human antibody (see, e.g., U.S. Patent Nos. 5,585,089, and No. 5,693,762; Jones et al., Nature, 321:522-525 (1986); Riechmann et al., 1988, Nature, 332:323-27 (1988); Verhoeyen et al., Science, 239:1534-1536 (1988)).

### 3. Class Hybrids

Antibodies of one subclass can be changed to antibodies from a different subclass using subclass switching methods. Thus, IgG antibodies may be derived from an IgM antibody, for example. Antibodies comprise, for example, variable domain combinations having a desired isotype (for example, IgA, IgG1, IgG2, IgG3, IgG4, IgE, and IgD). Such techniques allow the preparation of new antibodies with the antigen binding properties of the parent antibody and biological properties associated with an antibody isotype or subclass different from that of the parent antibody. See, e.g., Lantto et al., Methods Mol Biol, 178:303-316 (2002).

### 4. Chain Shuffling

Techniques for deriving antibodies having different properties (i.e., varying affinities for the antigen to which they bind) are also known. One such technique, referred to as chain shuffling, involves displaying immunoglobulin variable domain gene repertoires on the surface of filamentous bacteriophage (phage display). Chain shuffling has been used to prepare high affinity antibodies to the hapten 2-phenyloxazol-5-one, as described by Marks et al., BioTechnology, 10:779 (1992).

### D. Fully Human Antibodies

Fully human antibodies can be produced by introducing human immunoglobulin loci into mice in which the endogenous Ig genes have been inactivated. Thus, fully human antibodies can be produced by immunizing transgenic animals (usually mice) that are capable of producing a repertoire of human antibodies in the absence of endogenous immunoglobulin production. The preparation of such mice is described in detail in Taylor et al., Nucleic Acids Research, 20:6287-6295 (1992); Chen et al., International Immunology, 5:647-656 (1993); Tuaillon et al., J Immunol, 152:2912-2920 (1994); Lonberg, Handbook of Exp Pharmacology, 113:49-101 (1994); Taylor et al., International Immunology, 6:579-591 (1994); Fishwild et al., Nature Biotechnology, 14:845-85 (1996). See, further U.S. Patents 5,569,825; 5,625,126; 5,633,425; 5,789,650; 5,661,016; 5,814,318; and 5,770,429; PCT WO 93/1227; WO 92/22646; and WO 92/03918. Technologies utilized for producing human antibodies in these transgenic mice are disclosed also in PCT WO 98/24893, and Mendez et al., Nature Genetics, 15:146-156 (1997).

Fully human antibodies can also be derived from phage-display libraries (as disclosed in Hoogenboom et al., J Mol Biol, 227:381 (1991); and Marks et al., J Mol Biol, 222:581 (1991)). One such technique is described in PCT WO 99/10494.

Human antibodies can also be produced by exposing human splenocytes (B or T cells) to an antigen in vitro, then reconstituting the exposed cells in an immunocompromised mouse, e.g. SCID or nod/SCID. See Brams et al., J Immunol, 160:2051-2058 (1998); Carballido et al., Nat Med, 6:103-106 (2000). (McCune et al., Science, 241:1532-1639 (1988); Ifversen et al., Sem Immunol, 8:243-248 (1996)). In an alternative approach, human peripheral blood lymphocytes are transplanted intraperitoneally (or otherwise) into SCID mice (Mosier et al., Nature, 335:256-259 (1988); Martensson et al., Immunol, 84:224-230 (1995); and Murphy et al., Blood, 86:1946-1953 (1995)).

### E. Multispecific (Multifunctional) Antibodies

ANG-2 inhibitors also include multispecific antibodies that recognize more than one (e.g., two) epitopes on the same or different antigens. This can be done by V region or CDR grafting and/or multimerization. Multispecific antibodies may be produced by a variety of methods known in the art, including, but not limited to, fusion of hybridomas or chemical coupling of antibody fragments (e.g., Fab' fragments), see, e.g., Songsivilai and Lachmann, Clin. Exp. Immunol., 79:315-321 (1990); Kostelny et al., J Immunol, 148:1547-1553 (1992); U.S. Pat. No. 5,807,706; Cao and Suresh, Bioconjugate Chem, 9:635-644 (1998); Milstein and Cuello, Nature, 305:537-540 (1983); U.S. Pat. Nos. 4,474,893; 6,106,833; and 6,060,285; Brennan et al., Science, 229:81-83 (1985); Glennie et al., J Immunol, 139:2367-2375 (1987); and U.S. Pat. Nos. 6,010,902; 5,582,996; 5,959,083. In the present case, such antibodies can be prepared that (1) bind both ANG-2 and TIE2 (2) bind multiple ANG-2 and/or TIE2 epitopes or (3) bind multiple copies of ANG-2 and/or TIE2.

Moreover, single-chain variable fragments (sFvs) have been prepared by covalently joining two variable domains; the resulting antibody fragments can form dimers or trimers, depending on the length of a flexible linker between the two variable domains (Kortt et al., Protein Engineering, 10:423-433 (1997)).

### F. Single Chain Antibodies

Single chain antibodies may be formed by linking heavy and light chain variable domain (V_{L} and V_{H}) fragments by means of an amino acid bridge (short peptide linker), resulting in a single polypeptide chain. The resulting polypeptides can fold back on themselves to form antigen-binding monomers, or they can form multimers (e.g., dimers, trimers, or tetramers), depending on the length of a flexible linker between the two variable domains (Kortt et al., Prot Eng, 10:423 (1997); Kortt et al., Biomol Eng, 18:95-108 (2001)). By combining different V_{L} and V_{H} -comprising polypeptides, one can form multimeric scFvs that bind to different epitopes (Kriangkum et al., Biomol Eng, 18:31-40 (2001)). Techniques developed for the production of single chain antibodies include those described in U.S. Pat. No. 4,946,778; Bird, Science, 242:423 (1988); Huston et al., Proc Natl Acad Sci USA, 85:5879 (1988); Ward et al., Nature, 334:544 (1989), de Graaf et al., Methods Mol Biol, 178:379-387 (2002).

### G. Specific Antibody Inhibitors

The following references teach specific ANG-2 antibody inhibitors. These references are incorporated herein by reference for teaching these various ANG-2 inhibitors.

### Antibodies Against TIE2

U.S. Publications: 2007/0025993; 2006/0057138; 2006/0024297; U.S. Patents: 6,365,154; 6,376,653; PCT: WO 2006/020706; WO 2000/018437; WO 2000/018804.

### Antibodies Against TIE2 ECD

PCT WO1995/021866

### Antibodies Against ANG-2

U.S. Publications: 2006/0246071; 2006/0057138; 2006/0024297; 2006/0018909; 2005/0100906; 2003/0166858; 2003/0166857; 2003/0124129; 2003/0109677; 2003/0040463; 2002/0173627; U.S. Patents: 7,063,965; 7,063,840; 6,645,484; 6,627,415; 6,455,035; 6,433,143; 6,376,653; 6,166,185; 5,879,672; 5,814,464; 5,650,490; 5,643,755; 5,521,073; and PCT WO 2006/020706; WO 2006/045049; WO 2006/068953; WO 2003/030833.

Specific ANG-2 inhibitor antibodies include an antibody designated 536 (Tables 3 and 4) comprising a heavy chain and a light chain, where the heavy chain contains the variable region EVQLVQSGGGVVQPGRSLRLSCAASGFTFSSYGMHWVRQAPGK GLEWVSYISSSGSTIYYADSVKGRFTISRDNAKNSLYLQMNSLRAEDTAVYYCARDL LDYDILTGYGYWGQGTLVTVSS (SEQ ID NO: 41); and antigen binding fragments thereof; and the light chain comprises the variable region DIVMTQSPLSLPVTPGEPASI SCRSSQSLLHSNGYNFLDWYLQKPGQSPQLLIYLGSNRASGVPDRFSGSGSGTDFTLK ISRVEAEDVGVYYCMQGTHWPPTFGQGTKLEIK (SEQ ID NO: 42) or DIVMTQSPL SLPVTPGEPASISCRSSQSLLHSNGYNYLDWYLQKPGQSPQLLIYLGSNRASGVPDRF SGSGSGTDFTLKISRVEAEDVGVYYCMQGLQTPPTFGQGTKLEIK (SEQ ID NO: 43); and antigen binding fragments thereof, as described in U.S. 2006/045049, 2006/0018909 and 2003/0124129, and WO 03/030833.

Specific ANG-2 inhibitor antibodies also include an antibody designated H4L4 (Tables 1-4) comprising a heavy chain and a light chain, wherein the heavy chain comprises the heavy chain variable region H4 (SEQ ID NO: 3); and antigen binding fragments thereof; and the light chain comprises the light chain variable region L4 (SEQ ID NO: 10); and antigen binding fragments thereof. The H4 sequence is EVQLVQSGGGVVQPGRSLR LSCAASGFTFSSYGMHWVRQAPGKGLEWVSYISSSGSTIYYADSVKGRFTISRDNAK NSLYQMNSLRAEDTAVYYCARDLLDYDLLTGYGYWGQGTLVTVSS. The L4 sequence is DIVMTQSPLSLPVTPGEPASISCRSSQSLLHSHYNYLDWYLQKPGQSPQLL IYLGSNRASGVPDRFSGSGSGTDFTLKISRVEAEDVGVYYCMQGTHWPPTFGQGTKL EIK.

CDRs of both these ANG-2 inhibitors are described in Table 4.

### Generation of Randomized CDR-Based Peptides

To enhance the activity of ANG-2 antibody, CDR randomization was applied similar to previous approaches (Chen Y et al., J Mol Biol, 293:865-881 (1999); Yelton DE et al., J Immunol, 155:1994-2004 (1995); Yang W-P et al., J Mol Biol, 254:392-403 (1995)). Briefly, the variable regions of ANG-2 antibody 536 were cloned into the TargetQuest modified pCES-1 vector (Dyax Corp, de Haard HJ et al., JBiol Chem, 274:18218-30 (1999)). All CDR regions were targeted for randomization of each CDR residue by mutagenesis using NNK-containing oligonucleotides. After the mutagenesis reaction, phage clones were interrogated for each position using phage ELISA to identify beneficial mutations (for methods see WO 2004/046306,WO 2003/03057134, and US 2003/0099647 A1, for general phage antibody refs., Marks JD et al., J Mol Biol, 222:581-597 (1991); Hoogenboom HR et al., J Mol Biol, 227:381-388 (1992); Griffiths AD et al., EMBO J, 12:725-734 (1993); Vaughan TP et al., Nat Biotechnol, 14:309-314 (1996)). Clones with beneficial mutations were converted to full antibodies. Heavy chain clones were paired with light chain clones and the resulting IgG was tested for neutralization activity.

The following twenty-two antibodies were obtained. Each consisted of two heavy chains and two light (kappa or lambda) chains as designated in Table 1 below.

**Table 1**

| **Antibody*** | **Antibody Heavy Chain** | **Antibody Light Chain** |
|---|---|---|
| H6L7 | H6 HC | L7 LC |
| H5L7 | H5 HC | L7 LC |
| H4L13 | H4 HC | L13 LC |
| H11L7 | H11 HC | L7 LC |
| H10L7 | H10 HC | L7 LC |
| H4L7 | H4 HC | L7 LC |
| H5L6 | H5 HC | L6 LC |
| H2L7 | H2 HC | L7 LC |
| H5L8 | H5 HC | L8 LC |
| H6L8 | H6HC | L8 LC |
| H3L7 | H3 HC | L7 LC |
| H5L4 | H5HC | L4 LC |
| H4L12 | H4 HC | L12 LC |
| H6L6 | H6 HC | L6 LC |
| H4L2 | H4 HC | L2 LC |
| H4L6 | H4 HC | L6 LC |
| H4L4 | H4 HC | L4 LC |
| H5L11 | H5 HC | L11 LC |
| H5L1 | H5 HC | L1 LC |
| H4L11 | H4 HC | L11 LC |
| H5L12 | H5 HC | L12 LC |
| H5L9 | H5 HC | L9 LC |

| | | |
|---|---|---|
| * Tested for binding to hANG-2, mANG-2, and hANG-1. | | |

Tables 2 and 3 set forth the sequences and SEQ ID NOs. of the heavy and light (kappa and lambda) chains of the 22 anti-ANG-1 and/or anti-ANG-2 antibodies converted from phage to full length IgG1 antibodies. The CDRs of the monoclonal antibodies were predicted using the VBASE database which uses the technique described by Kabat et al in Sequences of Proteins of Immunological Interest (NIH Publication No. 91-3242; U.S. Dept. Health and Human Services, 5th ed.). Fab regions were aligned to sequences in the database with the closest germline sequence and then visually compared with such sequences. The CDRs for each variable region (heavy or light chain), both residue and sequences are set forth in Table 4.

**Table 2**

| **Heavy Chain Variable Regions** | |
|---|---|
| **Antibody HC** | **Sequence** |
| 536 HC (Ref) | EVQLVQSGGGVVQPGRSLRLSCAASGFTFSSYGMHWVRQAPGK GLEWVSYISSSGSTIYYADSVKGRFTISRDNAKNSLYLQMNSLRA EDTAVYYCARDLLDYDILTGYGYWGQGTLVTVSS |
| H2 (SEQ ID NO: 1) | EVQLVQSGGGVVQPGRSLRLSCAASGFTFSSYGMHWVRQAPGK GLEWVSYISSSGSTIEYADSVKGRFTISRDNAKNSLYLQMNSLRA EDTAVYYCARDLLDYDILTGYGYWGQGTLVTVSS |
| H3 (SEQ ID NO: 2) | EVQLVQSGGGVVQPGRSLRLSCAASGFTFSSYGMHWVRQAPGK GLEWVSYISSSGSTIQYADSVKGRFTISRDNAKNSLYLQMNSLRA EDTAVYYCARDLLDYDILTGYGYWGQGTLVTVSS |
| H4 (SEQ ID NO: 3) | EVQLVQSGGGVVQPGRSLRLSCAASGFTFSSYGMHWVRQAPGK GLEWVSYISSSGSTIYYADSVKGRFTISRDNAKNSLYLQMNSLRA EDTAVYYCARDLLDYDLLTGYGYWGQGTLVTVSS |
| H6 (SEQ ID NO: 4) | EVQLVQSGGGVVQPGRSLRLSCAASGFTFSSYGMHWVRQAPGK GLEWVSYISSSGSTIYYADSVKGRFTISRDNAKNSLYLQMNSLRA EDTAVYYCARDLLDYDIYTGYGYWGQGTLVTVSS |
| H10 (SEQ ID NO: 5) | EVQLVQSGGGVVQPGRSLRLSCAASGFTFSSYGMHWVRQAPGK GLEWVSYISSSGSTIYYADSVKGRFTISRDNAKNSLYLQMNSLRA EDTAVYYCARDLLDYDILTGYGLWGQGTLVTVSS |
| H11 (SEQ ID NO: 6) | EVQLVQSGGGVVQPGRSLRLSCAASGFTFSSYGMHWVRQAPGK GLEWVSYISSSGSTIYYADSVKGRFTISRDNAKNSLYLQMNSLRA EDTAVYYCARDLLDYDILTGYGMWGQGTLVTVSS |
| H5P (SEQ ID NO: 7) | EVQLVQSGGGVVQPGRSLRLSCAASGFTFSSYGMHWVRQAPGK GLEWVSYISSSGSTIYYADSVKGRFTISRDNAKNSLYLQMNSLRA EDTAVYYCARDLLDYDIWTGYGYWGQGTLVTVSS |

**Table 3**

| **Light Chain Variable Regions** | |
|---|---|
| **Antibody LC** | **Sequence** |
| 536 kappa (Ref) | DIVMTQSPLSLPVTPGEPASISCRSSQSLLHSNGYNYLDWYLQKP GQSPQLLIYLGSNRASGVPDRFSGSGSGTDFTLKISRVEAEDVGV YYCMQGTHWPPTFGQGTKLEIK |
| L1 (SEQ ID NO: 8) | DIVMTQSPLSLPVTPGEPASISCRSIQSLLQSNGYNYLDWYLQKP GQSPQLLIYLGSNRASGVPDRFSGSGSGTDFTLKISRVEAEDVGV YYCMQGTHWPPTFGQGTKLEIK |
| L2 (SEQ ID NO: 9) | DIVMTQSPLSLPVTPGEPASISCRSSQSLLLSNGYNYLDWYLQKP GQSPQLLIYLGSNRASGVPDRFSGSGSGTDFTLKISRVEAEDVGV YYCMQGTHWPPTFGQGTKLEIK |
| L4 (SEQ ID NO: 10) | DIVMTQSPLSLPVTPGEPASISCRSSQSLLHSHGYNYLDWYLQKP GQSPQLLIYLGSNRASGVPDRFSGSGSGTDFTLKISRVEAEDVGV YYCMQGTHWPPTFGQGTKLEIK |
| L6 (SEQ ID NO: 11) | DIVMTQSPLSLPVTPGEPASISCRSSQSLLHSVGYNYLDWYLQKP GQSPQLLIYLGSNRASGVPDRFSGSGSGTDFTLKISRVEAEDVGV YYCMQGTHWPPTFGQGTKLEIK |
| L7 (SEQ ID NO: 12) | DIVMTQSPLSLPVTPGEPASISCRSSQSLLHSNGYNFLDWYLQKP GQSPQLLIYLGSNRASGVPDRFSGSGSGTDFTLKISRVEAEDVGV YYCMQGTHWPPTFGQGTKLEIK |
| L8 (SEQ ID NO: 13) | DIVMTQSPLSLPVTPGEPASISCRSSQSLLHSNGYNMLDWYLQKP GQSPQLLIYLGSNRASGVPDRFSGSGSGTDFTLKISRVEAEDVGV YYCMQGTHWPPTFGQGTKLEIK |
| L9 (SEQ ID NO: 14) | DIVMTQSPLSLPVTPGEPASISCRSSQSLLHSNGYNYLDWYLQKP GQSPQLLIYAGSNRASGVPDRFSGSGSGTDFTLKISRVEAEDVGV YYCMQGTHWPPTFGQGTKLEIK |
| L11 (SEQ ID NO: 15) | DIVMTQSPLSLPVTPGEPASISCRSSQSLLHSNGYNYLDWYLQKP GQSPQLLIYLGSDRASGVPDRFSGSGSGTDFTLKISRVEAEDVGV YYCMQGTHWPPTFGQGTKLEIK |
| L12 (SEQ ID NO: 16) | DIVMTQSPLSLPVTPGEPASISCRSSQSLLHSNGYNYLDWYLQKP GQSPQLLIYLGSNRASGVPDRFSGSGSGTDFTLKISRVEAEDVGV YYCMQATHWPPTFGQGTKLEIK |
| L13 (SEQ ID NO: 17) | DIVMTQSPLSLPVTPGEPASISCRSSQSLLHSNGYNYLDWYLQKP GQSPQLLIYLGSNRASGVPDRFSGSGSGTDFTLKISRVEAEDVGV YYCMQVTHWPPTFGQGTKLEIK |

**Table 4**

| **Complementarity-Determining Regions (CDRs) of Heavy Chains (HC) and Light Chains (LC) of Ang-1 and /or Ang-2 Antibodies: Residues and Sequence** | | | | | | |
|---|---|---|---|---|---|---|
| | **CDR 1** | | **CDR 2** | | **CDR 3** | |
| **Antibody** | **Residues** | **Sequence** | **Residues** | **Sequence** | **Residues** | **Sequence** |
| Ab 536 HC | 31-35 | SYGMH | 50-66 | YISSSGSTIYYADSVKG | 99-111 | DLLDYDILTGYGY |
| Ab 536 LC | 24-39 | RSSQSLLHSNGYNYLD | 55-61 | LGSNRAS | 94-102 | MQGTHWPPT |
| H6L7 HC | 31-35 | SYGMH (SEQ ID NO: 18) | 50-66 | YISSSGSTIYYADSVKG (SEQ ID NO: 19) | 99-111 | DLLDYDI***Y***TGYGY (SEQ ID NO: 22) |
| H6L7 LC | 24-39 | RSSQSLLHSNGYN***F***LD (SEQ ID NO: 28) | 55-61 | LGSNRAS (SEQ ID NO: 35) | 94-102 | MQGTHWPPT (SEQ ID NO: 38) |
| H5L7 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L7 LC | 24-39 | RSSQSLLHSNGYN***F***LD (SEQ ID NO: 28) | 55-61 | LGSNRAS (SEQ ID NO: 35) | 94-102 | MQGTHWPPT (SEQ ID NO: 38) |
| H4L13 HC | 31-35 | SYGMH (SEQ ID NO: 18) | 50-66 | YISSSGSTIYYADSVKG (SEQ ID NO: 19) | 99-111 | DLLDYD***L***LTGYGY (SEQ ID NO: 24) |
| H4L13 LC | 24-39 | RSSQSLLHSNGYNYLD (SEQ ID NO: 29) | 55-61 | LGSNRAS (SEQ ID NO: 35) | 94-102 | MQ***V***THWPPT (SEQ ID NO: 39) |
| H11L7 HC | 31-35 | SYGMH (SEQ ID NO: 18) | 50-66 | YISSSGSTIYYADSVKG (SEQ ID NO: 19) | 99-111 | DLLDYDILTG***M***GY (SEQ ID NO: 25) |
| H11L7 LC | 24-39 | RSSQSLLHSNGYN***F***LD (SEQ ID NO: 28) | 55-61 | LGSNRAS (SEQ ID NO: 35) | 94-102 | MQGTHWPPT (SEQ ID NO: 38) |
| H10L7 HC | 31-35 | SYGMH (SEQ ID NO: 18) | 50-66 | YISSSGSTIYYADSVKG (SEQ ID NO: 19) | 99-111 | DLLDYDILTG***L***GY (SEQ ID NO: 26) |
| H10L7 LC | 24-39 | RSSQSLLHSNGYN***F***LD (SEQ ID NO: 28) | 55-61 | LGSNRAS (SEQ ID NO: 35) | 94-102 | MQGTHWPPT (SEQ ID NO: 38) |
| H4L7 HC | 31-35 | SYGMH (SEQ ID NO: 18) | 50-66 | YISSSGSTIYYADSVKG (SEQ ID NO: 19) | 99-111 | DLLDYD***L***LTGYGY (SEQ ID NO: 24) |
| H4L7 LC | 24-39 | RSSQSLLHSNGYN***F***LD (SEQ ID NO: 28) | 55-61 | LGSNRAS (SEQ ID NO: 35) | 94-102 | MQGTHWPPT (SEQ ID NO: 38) |
| H5L6 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L6 LC | 24-39 | RSSQSLLHS***V***GYNYLD (SEQ ID NO: 30) | 55-61 | LGSNRAS (SEQ ID NO: 35) | 94-102 | MQGTHWPPT (SEQ ID NO: 38) |
| H2L7 HC | 31-35 | SYGMH (SEQ ID NO: 18) | 50-66 | YISSSGSTI***E***YADSVKG (SEQ ID NO: 20) | 99-111 | DLLDYDILTGYGY (SEQ ID NO:27) |
| H2L7 LC | 24-39 | RSSQSLLHSNGYN***F***LD (SEQ ID NO: 28) | 55-61 | LGSNRAS (SEQ ID NO: 35) | 94-102 | MQGTHWPPT (SEQ ID NO: 38) |
| H5L8 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L8 LC | 24-39 | RSSQSLLHSNGYN***M***LD (SEQ ID NO: 31) | 55-61 | LGSNRAS (SEQ ID NO: 35) | 94-102 | MQGTHWPPT (SEQ ID NO: 38) |
| H6L8 HC | 31-35 | SYGMH (SEQ ID NO: 18) | 50-66 | YISSSGSTIYYADSVKG (SEQ ID NO: 19) | 99-111 | DLLDYDI***Y***TGYGY (SEQ ID NO: 22) |
| H6L8 LC | 24-39 | RSSQSLLHSNGYN***M***LD (SEQ ID NO: 31) | 55-61 | LGSNRAS (SEQ ID NO: 35) | 94-102 | MQGTHWPPT (SEQ ID NO: 38) |
| H3L7 HC | 31-35 | SYGMH (SEQ ID NO: 18) | 50-66 | YISSSGSTI***Q***YADSVKG (SEQ ID NO: 21) | 99-111 | DLLDYDILTGYGY (SEQ ID NO:27) |
| H3L7 LC | 24-39 | RSSQSLLHSNGYN***F***LD (SEQ ID NO: 28) | 55-61 | LGSNRAS (SEQ ID NO: 35) | 94-102 | MQGTHWPPT (SEQ ID NO: 38) |
| H5L4 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L4 LC | 24-39 | RSSQSLLHS***H***GYNYLD (SEQ ID NO: 32) | 55-61 | LGSNRAS (SEQ ID NO: 35) | 94-102 | MQGTHWPPT (SEQ ID NO: 38) |
| H4L12 HC | 31-35 | SYGMH (SEQ ID NO: 18) | 50-66 | YISSSGSTIYYADSVKG (SEQ ID NO: 19) | 99-111 | DLLDYD***L***LTGYGY (SEQ ID NO: 24) |
| H4L12 LC | 24-39 | RSSQSLLHSNGYNYLD (SEQ ID NO: 29) | 55-61 | LGSNRAS (SEQ ID NO: 35) | 94-102 | MQ***A***THWPPT (SEQ ID NO: 40) |
| H6L6 HC | 31-35 | SYGMH (SEQ ID NO: 18) | 50-66 | YISSSGSTIYYADSVKG (SEQ ID NO: 19) | 99-111 | DLLDYDI***Y***TGYGY (SEQ ID NO: 22) |
| H6L6 LC | 24-39 | RSSQSLLHS***V***GYNYLD (SEQ ID NO: 30) | 55-61 | LGSNRAS (SEQ ID NO: 35) | 94-102 | MQGTHWPPT (SEQ ID NO: 38) |
| H4L2 HC | 31-35 | SYGMH (SEQ ID NO: 18) | 50-66 | YISSSGSTIYYADSVKG (SEQ ID NO: 19) | 99-111 | DLLDYD***L***LTGYGY (SEQ ID NO: 24) |
| H4L2 LC | 24-39 | RSSQSLL***L***SNGYNYLD (SEQ ID NO: 33) | 55-61 | LGSNRAS (SEQ ID NO: 35) | 94-102 | MQGTHWPPT (SEQ ID NO: 38) |
| H4L6 HC | 31-35 | SYGMH (SEQ ID NO: 18) | 50-66 | YISSSGSTIYYADSVKG (SEQ ID NO: 19) | 99-111 | DLLDYD***L***LTGYGY (SEQ ID NO: 24) |
| H4L6 LC | 24-39 | RSSQSLLHS***V***GYNYLD (SEQ ID NO: 30) | 55-61 | LGSNRAS (SEQ ID NO: 35) | 94-102 | MQGTHWPPT (SEQ ID NO: 38) |
| H4L4 HC | 31-35 | SYGMH (SEQ ID NO: 18) | 50-66 | YISSSGSTIYYADSVKG (SEQ ID NO: 19) | 99-111 | DLLDYD***L***LTGYGY (SEQ ID NO: 24) |
| H4L4 LC | 24-39 | RSSQSLLHS***H***GYNYLD (SEQ ID NO: 32) | 55-61 | LGSNRAS (SEQ ID NO: 35) | 94-102 | MQGTHWPPT (SEQ ID NO: 38) |
| H5L11 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L11 LC | 24-39 | RSSQSLLHSNGYNYLD (SEQ ID NO: 29) | 55-61 | LGSDRAS (SEQ ID NO: 36) | 94-102 | MQGTHWPPT (SEQ ID NO: 38) |
| H5L1 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L1 LC | 24-39 | RS***I***QSLL***Q***SNGYNYLD (SEQ ID NO: 34) | 55-61 | LGSNRAS (SEQ ID NO: 35) | 94-102 | MQGTHWPPT (SEQ ID NO: 38) |
| H4L11 HC | 31-35 | SYGMH (SEQ ID NO: 18) | 50-66 | YISSSGSTIYYADSVKG (SEQ ID NO: 19) | 99-111 | DLLDYD***L***LTGYGY (SEQ ID NO: 24) |
| H4L11 LC | 24-39 | RSSQSLLHSNGYNYLD (SEQ ID NO: 29) | 55-61 | LGSDRAS (SEQ ID NO: 36) | 94-102 | MQGTHWPPT (SEQ ID NO: 38) |
| H5L12 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L12 LC | 24-39 | RSSQSLLHSNGYNYLD (SEQ ID NO: 29) | 55-61 | LGSNRAS (SEQ ID NO: 35) | 94-102 | MQ***A***THWPPT (SEQ ID NO: 40) |
| H5L9 HC | 31-35 | SYGMH (SEQ ID NO: 18) | 50-66 | YISSSGSTIYYADSVKG (SEQ ID NO: 19) | 99-111 | DLLDYDI***W***TGYGY (SEQ ID NO: 23) |
| H5L9 LC | 24-39 | RSSQSLLHSNGYNYLD (SEQ ID NO: 29) | 55-61 | ***A***GSNRAS (SEQ ID NO: 37) | 94-102 | MQGTHWPPT (SEQ ID NO: 38) |

### 1gG Heavy Chain Constant Domain Isotype Sequence Information

The following sequences of the IgG1, IgG2, IgG3, and IgG4 isotypes can be used in combination with variable heavy chain sequences to make a specific desired isotype.

### Human IgG1 (SEQ ID NO: 49)

### Human IgG2 (SEQ ID NO: 50)

ASTKGPSVFPLAPCSRSTSESTAALGCLVKDYFPEPVTVSWNSGALTSGVH
TFPAVLQSSGLYSLSSVVTVPSSNFGTQTYTCNVDHKPSNTKVDKTVERKCCVECPP
CPAPPVAGPSVFLFPPKPKDTLMISRTPEVTCVVVDVSHEDPEVQFNWYVDGVEVHN
AKTKPREEQFNSTFRVVSVLTVVHQDWLNGKEYKCKVSNKGLPAPIEKTISKTKGQP
REPQVYTLPPSREEMTKNQVSLTCLVKGFYPSDIAVEWESNGQPENNYKTTPPMLDS
DGSFFLYSKLTVDKSRWQQGNVFSCSVMHEALHNHYTQKSLSLSPGK

### Human IgG3 (SEQ ID NO: 51)

ASTKGPSVFPLAPCSRSTSGGTAALGCLVKDYFPEPVTVSWNSGALTSGV
HTFPAVLQSSGLYSLSSVVTVPSSSLGTQTYTCNVNHKPSNTKVDKRVELKTPLGDT
THTCPRCPEPKSCDTPPPCPRCPEPKSCDTPPPCPRCPEPKSCDTPPPCPRCPAPELLGG

PSVFLFPPKPKDTLMISRTPEVTCVVVDVSHEDPEVQFKWYVDGVEVHNAKTKPREE
QYNSTFRVVSVLTVLHQDWLNGKEYKCKVSNKALPAPIEKTISKTKGQPREPQVYTL
PPSREEMTKNQVSLTCLVKGFYPSDIAVEWESSGQPENNYNTTPPMLDSDGSFFLYS
KLTVDKSRWQQGNIFSCSVMHEALHNRFTQKSLSLSPGK

### Human IgG4 (SEQ ID NO: 52)

ASTKGPSVFPLAPCSRSTSESTAALGCLVKDYFPEPVTVSWNSGALTSGVH
TFPAVLQSSGLYSLSSVVTVPSSSLGTKTYTCNVDHKPSNTKVDKRVESKYGPPCPSC
PAPEFEGGPSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTISKAKGQP
REPQVYTLPPSQEEMTKNQVSLTCLVKGFYPSDIAVEWESNGQPENNYKTTPPVLDS
DGSFFLYSRLTVDKSRWQEGNVFSCSVMHEALHNHYTQKSLSLSLGK

### Light Chain Constant Domain Sequence Information

### Kappa Light Constant Domain Protein (SEQ ID NO: 53):

### Lambda Light Constant Domain Protein (SEQ ID NO: 54):

GQPKAAPSVTLFPPSSEELQANKATLVCLISDFYPGAVTVAWKADSSPVK
AGVETTTPSKQSNNKYAASSYLSLTPEQWKSHRSYSCQVTHEGSTVEKTVAPTECS

### H4: An Example of the Combination of Heavy Chain Variable and Constant Region

The following sequence represents the H4 heavy chain sequence as IgG2 isotype. The underlined sequence portion represents the IgG2 sequence.

### H4 (SEQ ID NO: 55)

EVQLVQSGGGVVQPGRSLRLSCAASGFTFSSYGMHWVRQAPGKGLEWV
SYISSSGSTIYYADSVKGRFTISRDNAKNSLYLQMNSLRAEDTAVYYCARDLLDYDL
LTGYGYWGQGTLVTVSSASTKGPSVFPLAPCSRSTSESTAALGCLVKDYFPEPVTVS
WNSGALTSGVHTFPAVLQSSGLYSLSSVVTVPSSNFGTQTYTCNVDHKPSNTKVDKT
VERKCCVECPPCPAPPVAGPSVFLFPPKPKDTLMISRTPEVTCVVVDVSHEDPEVQFN

WYVDGVEVHNAKTKPREEQFNSTFRVVSVLTVVHQDWLNGKEYKCKVSNKGLPAP
IEKTISKTKGQPREPQVYTLPPSREEMTKNQVSLTCLVKGFYPSDIAVEWESNGQPEN
NYKTTPPMLDSDGSFFLYSKLTVDKSRWQQGNVFSCSVMHEALHNHYTQKSLSLSP
GK

### II. Peptides That Bind ANG-2

ANG-2 inhibitors also include peptides that bind to ANG-2 but which are not based on binding by means of Ig variable region sequences. These include TIE2 receptor-based peptides as well as peptides that are not TIE2 receptor-based.

### A. TIE2 Receptor-Based

### 1. Whole Soluble Receptor

In some embodiments, the inhibitor comprises the entire receptor or variants or derivatives of the receptor. Variants include naturally-occurring variants, such as allelic variants or naturally-occurring mutants. Variants also include manufactured variants produced by directed amino acid substitution, insertion or deletion.

### 2. Extracellular Domain

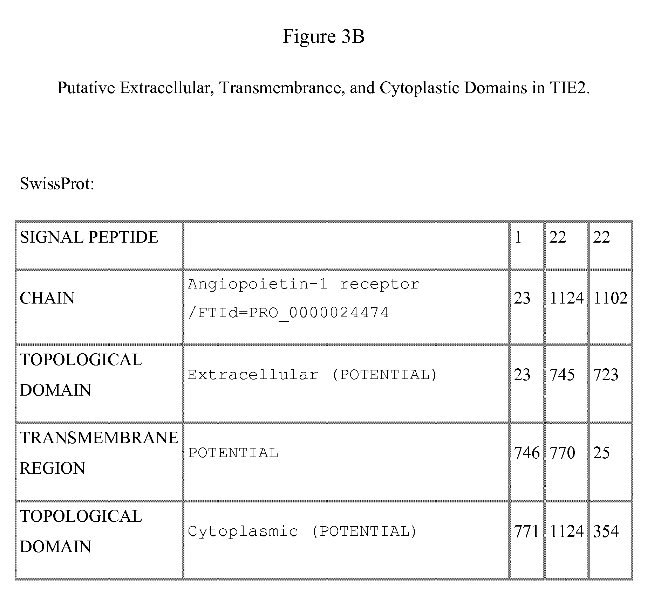

In some embodiments, ANG-2 inhibitor peptides can be based on the TIE2 extracellular domain (ECD). The native sequence of the full length TIE2 extracellular domain is set forth as residues 23-745 of SEQ ID NO: 46 and see Figure 3. Thus, all or part of the transmembrane domain and/or all or part of the cytoplasmic domain can be lacking. These polypeptides based on the ECD are intended to encompass fragments, variants, and derivatives of the ECD.

Peters et al. have described a soluble Tek (TIE2) inhibitor designated ExTek6His, consisting of the entire extracellular portion of murine TIE2 fused to a six-histidine tag (J Clin Invest, 199:2072 (1997); WO 98/18914). Peters et al. have also described a replication-defective adenoviral vector designated AdExTek, which expresses the murine Tek extracellular domain *(*Proc Natl Acad Sci USA 95:8829 (1998); WO 98/18914). Such inhibitors as the human ortholog are incorporated herein by reference. Another reference disclosing ANG-2 inhibitor peptides based on the ECD of TIE2 includes WO 00/75323, which describes polypeptides comprising a fragment of the TIE2 ECD. The fragment lacks all or part of the region containing the fibronectin type III (FNIII) motifs. The polypeptide retains the ability to bind at least one TIE2 ligand. A specific embodiment is designated "Tek472/Fc". It is a fusion of the N-terminal 472 amino acids of TIE2 to a 232 amino acid portion of the Fc region of human IgGl, as described in WO 00/75323A1, which is herein incorporated by reference for teaching Tek472/Fc, its structure and properties, methods for making and using it, and other related fusion polypeptides. The N-terminal TIE2 fragment constitutes a soluble TIE2 peptide that is missing fibronectin type III motifs. It has a much higher affinity for TIE2 ligands than other TIE2 polypeptides that comprise full-length extracellular domains. Other examples include embodiments where the ANG-2 inhibitor is a soluble TIE2 multimer, for example a dimer or trimer, and may also comprise an Fc polypeptide or a leucine zipper, as also described in WO 00/75323.

### B. Randomized TIE2 Peptides

In one embodiment, peptides comprise randomized TIE2 sequences. The term "randomized" with respect to peptide sequences refers to fully random sequences and sequences in which one or more residues of a naturally-occurring molecule is replaced by an amino acid residue not appearing in that position in the naturally-occurring molecule. Exemplary methods for identifying peptide sequences include phage display, E. coli display, ribosome display, RNA-peptide screening, chemical screening, and the like. Phage display, in particular, is useful in generating peptides for use in the present invention as has been shown that affinity selection from libraries of random peptides can be used to identify peptide ligands for any site of any gene product.

For phage display technology, see Dedman et al., J Biol Chem, 268:23025-30 (1993); Scott et al., Science, 249:386 (1990); Devlin et al., Science, 249: 404 (1990); U.S. Patent Nos. 5,223,409; 5,733,731; 5,498,530; 5,432,018; 5,338,665; 5,922,545; and WO 96/40987 and WO 98/15833 (all incorporated by reference). Cwirla et al., Science, 276:1696-9 (1997). The obtained peptide sequences may also suggest which residues may be safely replaced by alanine scanning or by mutagenesis at the DNA level. Mutagenesis libraries may be created and screened to further optimize the sequence of the best binders. (Lowman, Ann. Rev. Biophys Biomol Struct, 26:401-24 (1997)).

Alternatively, a peptide library can be fused to the carboxyl terminus of the lac repressor and expressed in E. coli. Another E. coli-based method allows display on the cell's outer membrane by fusion with a peptidoglycan-associated lipoprotein (PAL). These and related methods are collectively referred to as "E. coli display." In another method, translation of random RNA is halted prior to ribosome release, resulting in a library of polypeptides with their associated RNA still attached. This and related methods are collectively referred to as "ribosome display." Other methods employ chemical linkage of peptides to RNA. See, for example, Roberts and Szostak, Proc Natl Acad Sci USA, 94: 12297-303 (1997). This and related methods are collectively referred to as "RNA-peptide screening." Chemically derived peptide libraries have been developed in which peptides are immobilized on stable, non-biological materials, such as polyethylene rods or solvent-permeable resins. Another chemically derived peptide library uses photolithography to scan peptides immobilized on glass slides. Hereinafter, these and related methods are collectively referred to as "chemical-peptide screening." Chemical-peptide screening may be advantageous in that it allows use of D-amino acids and other unnatural analogues, as well as non-peptide elements. Both biological and chemical methods are reviewed in Wells and Lowman, Curr Opin Biotechnol, 3:355-62 (1992).

Structural analysis of protein-protein interaction may also be used to suggest peptides that mimic the binding activity of large protein ligands. In such an analysis, the crystal structure may suggest the identity and relative orientation of critical residues of the large protein ligand, from which a peptide may be designed. See, e.g., Takasaki et al., Nature Biotech, 15:1266-70 (1997)). These analytical methods may also be used to investigate the interaction between a receptor protein and peptides selected by phage display, which may suggest further modification of the peptides to increase binding affinity. For example, the structure of TIE2 may be used to create inhibitors of TIE2, as described in WO 2002/020734, U.S. 2003/0082622, and U.S. Patent No. 7,369,946, all of which are incorporated herein for teaching these inhibitors.

For methods of epitope mapping, for identification of critical amino acids in protein-protein interactions, and as leads for the discovery of new therapeutic agents, also see, Cortese et al., Curr Opin Biotech, 7:616-21 (1996). Peptide libraries are now being used most often in immunological studies, such as epitope mapping. See Kreeger, The Scientist, 10(13):19-20(1996).

### C. Specific ANG-2 Binding Peptide Inhibitors

The following references describe peptide inhibitors that bind ANG-2. These references are incorporated by reference for teaching these specific peptide inhibitors.

### Peptides Derived from TIE2

U.S. Patent 5,681,714; PCT: WO 2004/076650; WO 2000/065085; and WO 1995/021866.

### Peptides Derived from ECD of TIE2

U.S. Publications: 2007/0025993; 2005/0100906; 2003/0166858; 2003/0166857; 2003/0162712; 2003/0109677; 2003/0040463; 2002/0173627; 2002/0039992; U.S. Patents: 7,067,475; 7,063,965; 7,063,840; 6,645,484; 6,627,415; 6,521,424; 6,433,143; 6,413,932; 5,879,672; 5,814,464; 5,650,490; 5,643,755; 5,521,073; 6,413,932; PCT: WO/2000/075323; WO/2006/002854; WO/1998/018914.

### Peptides That Bind ANG-2 - Not Based on TIE2 (Randomly Generated)

2007/0225221; 2007/0093419; 2007/0093418; 2007/0072801; 2006/0122370; 2003/0236193; 2003/0229023; 7,205,275; 7,138,370; WO 2004/092215; WO 2003/057134. Specifically, ANG-2 inhibitor peptides L1-7 and L1-10 are described in WO 2004/092215 and WO 2003/057134, which are incorporated herein in their entirety for teaching ANG-2 peptide inhibitors. L1-7 is TNFMPMDDLEQRLYEQFILQQG (SEQ ID NO: 44) and L1-10 is QKFQPLDELEQTLYEQFMLQQA (SEQ ID NO: 45).
In another embodiment the Ang-2 inhibitor peptide is 2XCon(4)C (SEQ ID NO: 56) (identified as SEQ ID NO: 25 of US Patent No. 7,138,370, incorporated herein by reference), wherein the sequence is:
M-Fc-GGGGGAQQEECEWDPWTCEHMGSGSATGGSGSTASSGSGSATHQEECEWDPWTC EHMLE (SEQ ID NO: 56). In a particular embodiment, the Fc region of the 2XCon(4)C is an IgG1 Fc (such as, but not limited to, SEQ ID NO: 60 of US Patent No. 7,138,370, incorporated herein by reference), wherein the sequence is

DKTHTCPPCPAPELLGGPSVFLFPPKPKDTLMISRTPEVTCVVVDV
SHEDPEVKFNWYVDGVEVHNAKTKPREEQYNSTYRVVSVLTVL
HQDWLNGKEYKCKVSNKALPAPIEKTISKAKGQPREPQVYTLPPS
RDELTKNQVSLTCLVKGFYPSDIAVEWESNGQPENNYKTTPPVL
DSDGSFFLYSKLTVDKSRWQQGNVFSCSVMHEALHNHYTQKSLS
LSPGK   (SEQ ID NO: 57).

### III. Peptides That Bind TIE2

### A. ANG-2 Based Peptide Inhibitors

Inhibitors also include peptides that bind to TIE2. Such peptides can be based on the ANG-2 protein itself. The native sequence of the full length ANG-2 is set forth in Figure 3C. However, ANG-2-based inhibitor proteins may comprise variants or fragments of the native ANG-2 sequence. Accordingly, useful ANG-2 variants for the purpose of the present invention include, but are not limited to, the following (1) variants that compete with endogenous ANG-2 by binding to the TIE2 receptor but which bind in such a way that the receptor is not inactivated (auto-phosphorylation is not achieved). Such variants may have a higher affinity for the TIE2 receptor.

The invention includes specific variants. Kim et al. (2000 supra) isolated a cDNA encoding a splice variant of ANG-2, termed ANG-2(443) because the 443-amino acid protein lacks 53 amino acids found in the full-length ANG-2 protein. The missing residues, amino acids 96 to 148, are encoded by exon B of the ANG-2 gene and include part of the N-terminal coiled-coil domain. Binding analysis demonstrated that ANG-2(443), like ANG-2 and ANG-1, binds TIE2 but not TIE1. The truncated ANG-2(443) protein partially inhibits ANG-2 and ANG-1 binding to TIE2 as well as ANG-1-induced phosphorylation of TIE2.

In a further embodiment, TIE2 binding peptides comprise randomized ANG-2 sequences, as discussed above for peptides that bind ANG-2.

### B. Specific ANG-2 Based TIE2 Binding Peptide Inhibitors

The following references disclose specific peptides that bind to TIE2. These are incorporated by reference for these specific peptide inhibitors.

### Peptides Derived From ANG-2

U.S. Publications: 2005/0186665; 2005/0175617; 2005/0106099; 2005/0100906; 2003/0166858; 2003/0166857; 2003/0109677; 2003/0092891; 2003/0040463; 2002/0173627; U.S. Patents: 7,309,483; 7,063,965; 7,063,840; 7,045,302; 6,825,008; 6,645,484; 6,627,415; 6,441,137; 6,433,143; 6,265,564; 5,879,672; 5,814,464; 5,650,490; 5,643,755; 5,521,073

### Peptides That Bind TIE2 - Not Based on ANG-2 (Randomly Generated)

U.S. Patent Nos. 7,008,781; 6,455,035; and 6,166,185; PCT W02006/005361.

### IV. Vehicles

Any of the peptides and proteins described above can be attached to a vehicle. Vehicles may be another protein or peptide or may be non-proteinaceous. The term "vehicle" refers to a molecule that prevents degradation and/or increases half-life (e.g., by avoiding sequences recognized by proteases), reduces toxicity, reduces immunogenicity (e.g., by favoring non-immunogenic sequences), or increases biological activity of a therapeutic protein. Exemplary vehicles include an 1g Fc domain as well as a linear polymer (e.g., polyethylene glycol (PEG), polylysine, dextran, etc.); a branched-chain polymer (See, for example, U.S. Patent No. 4,289,872; U. S. Patent No. 5,229,490; WO 93/21259); a lipid; a cholesterol group (such as a steroid); a carbohydrate or oligosaccharide; or any natural or synthetic protein, polypeptide or peptide. For polymer vehicles, various means for attaching chemical moieties useful as vehicles are currently available, see, e.g., WO 96/11953, herein incorporated by reference for disclosing the selective attachment of water soluble polymers to the N-terminus of proteins.

Thus, at least one peptide can be attached to at least one vehicle through the N-terminus, C-terminus or a side chain of one of the amino acid residues of the peptide(s). Multiple same or different vehicles may also be used, such as at each terminus or an Fc at a terminus and/or a side chain. In one embodiment, the vehicle is the Fc portion of an antibody to form a "peptibody". Examples of suitable ANG-2 inhibitor peptibodies are described in U.S. Patent Nos. 6,413,932; 7,138,370; and 7,205,275; U.S. Publication Nos. 20070072801, 20070093418, 20070093419, 20070225221, and 20060122370, and PCT application Nos. WO 2004/092215 and WO 2003/057134, each of which is incorporated by reference herein for teaching the ANG-2 inhibitor peptibodies.

Fc variants are also suitable vehicles. See, for example WO 97/34631 and WO 96/32478. One suitable Fc polypeptide, described in PCT WO 93/10151 and U.S. Patent. No. 5,426,048 and No. 5,262,522, is a single chain polypeptide extending from the N-terminal hinge region to the native C-terminus of the Fc region of a human IgG1 antibody. Another useful Fc polypeptide is the Fc mutein described in U.S. Patent No. 5,457,035, and in Baum et al., EMBO J, 13:3992-4001 (1994). The amino acid sequence of this mutein is identical to that of the native Fc sequence presented in PCT WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors.

### V. Multimers

In one embodiment, any of the ANG-2 protein or peptide inhibitors are covalently-linked or non-covalently-linked to form multimers, including dimers, trimers, or higher multimers. Multimers may be linked by disulfide bonds formed between cysteine residues on different polypeptides by peptide linkers (spacers), or by peptides that have the property of promoting multimerization (such as leucine zippers and certain polypeptides derived from antibodies). In particular embodiments, the multimers comprise from two to four soluble TIE2 polypeptides. Suitable peptide linkers are those described in U.S. Pat. Nos. 4,751,180, 4,935,233, and 5,073,627. Suitable leucine zippers are described in Landschulz et al., Science 240:1759, (1988), PCT application WO 94/10308, and the leucine zipper derived from lung surfactant protein D (SPD) described in Hoppe et al. (FEBS Lett, 344:191, (1994)).

In some embodiments, multimers are prepared using polypeptides derived from immunoglobulins. Preparation of fusion proteins comprising certain heterologous polypeptides fused to various portions of antibody-derived polypeptides (including the Fc domain) has been described, e.g., by Ashkenazi et al. (Proc Natl Acad Sci USA, 88:10535, (1991)); Byrn et al. (Nature, 344:677, 1990)); and Hollenbaugh and Aruffo ("Construction of Immunoglobulin Fusion Proteins", in Current Protocols in Immunology, Suppl. 4, pp. 10.19.1-10.19.11, 1992)). Thus, for example, peptibodies can be multimerized by interchain disulfide bonds formed between the Fc moieties.

### VI. Methods of Making Peptides

ANG-2 peptide or protein inhibitors can be generated using a wide variety of techniques known in the art. For example, such peptides can be synthesized. Various automatic synthesizers are commercially available and can be used in accordance with known protocols. See, for example, Stewart and Young (supra); Tam et al., J Am Chem Soc, 105:6442, (1983); Merrifield, Science, 232:341-347 (1986); Barany and Merrifield, The Peptides, Gross and Meienhofer, eds, Academic Press, New York, 1-284; Barany et al., Int J Pep Protein Res, 30:705-739 (1987); and U.S. Patent No. 5,424,398, each incorporated herein by reference. They may also be produced by standard recombinant DNA techniques.

### VII. Modifications of Peptides (Derivatives)

The invention also encompasses derivatives of any of the peptide or protein inhibitors. The primary amino acid structure of any of the ANG-2 inhibitors may be modified to create derivatives by forming covalent or aggregative conjugates with other chemical moieties, such as glycosyl groups, lipids, phosphate, acetyl groups and the like. Covalent derivatives may be prepared by linking particular functional groups to amino acid side chains or at the N-terminus or C-terminus of the polypeptide.

Such derivatized moieties may increase the suitability of the protein for a particular use (e.g., administration to a subject, such as a human subject, or other *in vivo* or *in vitro* uses) and preferably improve one or more characteristics including anti-inflammation activity, solubility, absorption, circulating half-life, improved targeting capacity to desired cells, tissue, or organs, and the like. Alternatively, derivatized moieties may result in compounds that have the same, or essentially the same, characteristics and/or properties of the compound that is not derivatized, but eliminates or attenuates any undesirable side effect. Preferably, the modifications are covalent in nature, and include for example, chemical bonding with polymers, lipids, other organic, and inorganic moieties.

Examples of molecules that can be used to derivatize an antigen binding protein include albumin (e.g., human serum albumin) and polyethylene glycol (PEG). Albumin-linked and PEGylated derivatives of antigen binding proteins can be prepared using techniques well known in the art. In one embodiment, the antigen binding protein is conjugated or otherwise linked to transthyretin (TTR) or a TTR variant. The TTR or TTR variant can be chemically modified with, for example, a chemical selected from the group consisting of dextran, poly(n-vinyl pyrrolidone), polyethylene glycols, propropylene glycol homopolymers, polypropylene oxide/ethylene oxide co-polymers, polyoxyethylated polyols and polyvinyl alcohols.

Other possible modifications include hydroxylation of proline and lysine, phosphorylation of hydroxyl groups of seryl or threonyl residues, oxidation of the sulfur atom in Cys, methylation of the alpha-amino groups of lysine, arginine, and histidine side chains (Creighton, T.E., Proteins: Structure and Molecule Properties, W. H. Freeman & Co., San Francisco, pp. 79-86 (1983)), acetylation of the N-terminal amine, and, in some instances, amidation of the C-terminal carboxyl groups.

In certain embodiments, ANG-2 inhibitor derivatives include glycosylation variants wherein one or more glycosylation sites, such as a N-linked glycosylation site, has been deleted, added, or rearranged. This may be done by standard recombinant DNA techniques (i.e., DNA glycosylation sequences As-X-Ser or Asn-X-Thr) or chemically. These methods are described in PCT Publication No. WO 87/05330, and in Aplin and Wriston, CRC Crit Rev Biochem, 259-306 (1981). Removal of carbohydrate moieties present on the ANG-2 inhibitor may be accomplished chemically or enzymatically. Chemical deglycosylation is described by Hakimuddin et al., Arch Biochem Biophys, 259:52 (1987) and by Edge et al., Anal Biochem, 118:131 (1981). Enzymatic cleavage is described by Thotakura et al., Meth Enzymol, 138:350 (1987). Glycosylation at potential glycosylation sites may be prevented by the use of the compound tunicamycin as described by Duskin et al., J Biol Chem, 257:3105 (1982). Tunicamycin blocks the formation of protein-N-glycoside linkages.

A preferred polymer vehicle is polyethylene glycol (PEG). The PEG group may be of any convenient molecular weight and may be linear or branched. The average molecular weight of the PEG will preferably range from about 2 kiloDalton ("kDa") to about 100 kDa, more preferably from about 5 kDa to about 50 kDa, most preferably from about 5 kDa to about 10 kDa. The PEG groups will generally be attached to the compounds of the invention via acylation or reductive alkylation through a reactive group on the PEG moiety (e.g., an aldehyde, amino, thiol, or ester group) to a reactive group on the inventive compound (e.g., an aldehyde, amino, or ester group).

Polysaccharide polymers are another type of water soluble polymer which may be used for protein modification. Dextrans are polysaccharide polymers comprised of individual subunits of glucose predominantly linked by al-6 linkages. The dextran itself is available in many molecular weight ranges, and is readily available in molecular weights from about 1 kDa to about 70 kDa. Dextran is a suitable water-soluble polymer for use in the present invention as a vehicle by itself or in combination with another vehicle (e.g., Fc). See, for example, WO 96/11953 and WO 96/05309. The use of dextran conjugated to therapeutic or diagnostic immunoglobulins has been reported; see, for example, European Patent Publication No. 0 315 456, which is hereby incorporated by reference. Dextran of about 1 kDa to about 20 kDa is preferred when dextran is used as a vehicle in accordance with the present invention.

### VIII. Screening/Binding Assays

Screening procedures by which antibody and other peptide inhibitors can be identified are well-known and can involve physical assays, such as affinity chromatography, for example. Or these may be combined with biological assays such as binding, phosphorylation, or effect on signal transduction, such as in reporter-based assays.

Various immunological binding assays, as known to one of ordinary skill in the art, may be used to identify and/or assess ANG-2 antibody inhibitors, for example, the assays described in WO 2004/092215 and WO 2000/057134 and Asai, ed., Methods in Cell Biology, Vol. 37, Antibodies in Cell Biology, Academic Press, Inc., New York (1993).

Numerous types of competitive binding assays can be used, for example: solid phase direct or indirect radioimmunoassay (RIA), solid phase direct or indirect enzyme immunoassay (EIA), sandwich competition assay (see, e.g., Stahli et al., Methods in Enzymology, 9:242-253 (1983)); solid phase direct biotin-avidin EIA (see, e.g., Kirkland et al., J Immunol, 137:3614-3619 (1986)) solid phase direct labeled assay, solid phase direct labeled sandwich assay (see, e.g., Harlow and Lane, Antibodies, A Laboratory Manual, Cold Spring Harbor Press (1998)); solid phase direct label RIA using I-125 label (see, e.g., Morel et al., Molec Immunol 25:7-15 (1988)); solid phase direct biotin-avidin EIA (see, e.g., Cheung, et al., Virology 176:546-552 (1990)); and direct labeled RIA (Moldenhauer et al., Scand J Immunol, 32:77-82 (1990)).

Immunological binding assays can be of the non-competitive type. These assays have an amount of captured analyte that is directly measured. Immunological binding assays can be of the competitive type. The amount of analyte present in the sample is measured indirectly by measuring the amount of an added analyte displaced, or competed away, from a capture agent, for example, antibody or peptibody, by the analyte present in the sample. The competitive binding assays can be used for cross-reactivity determinations to permit a skilled artisan to determine if a protein or enzyme complex which is recognized by, for example, a peptibody of the invention, is the desired protein and not a cross-reacting molecule or to determine whether the peptibody is specific for the antigen and does not bind unrelated antigens. See Harlow and Lane, Antibodies, A Laboratory Manual, Ch 14, Cold Spring Harbor Laboratory, NY (1988), incorporated herein by reference.

### IX. Small Molecules that Inhibit ANG-2 Binding and/or TIE2 Activation by ANG-2

ANG-2 inhibitors of the present invention can also be small molecules. For example, small molecule inhibitors of TIE2 and/or ANG-2 have been described. The following examples of small molecule ANG-2 inhibitors are incorporated by reference herein for the disclosure of ANG-2 inhibitors: [Pfizer] - U.S. Publication Nos. 20060035912 and 20050037999; [Cephalon] - U.S. Publication No. 20070203135; [Bayer] - U.S. Publication Nos. 20080064707, 20080064696, 20080058326, 20080039482, 20060194823, 20060252754, and 20060167030; [Schering Aktiengesellschaft] - U.S. Publication No. 20060252782, PCT Publication Nos. WO 06/108695, WO 06/077168, WO 06/066957, WO 06/066956, WO 08/006560, WO 07/147575, WO 07/147574, WO 07/144204, WO 07/144203, and WO 07/144202; [Astrazeneca] - U.S. Publication Nos. 20080027076, 20070135455, 20060069109, 20050256140, PCT Publication Nos. WO 06/103449, WO 06/082404, WO 06/082371, WO 05/075483, WO 05/060970, WO 05/060969, WO 04/013141, and WO 04/058776; [GlaxoSmithKline] - U.S. Patent Nos. 7,338,959, and 7,238,813, 7,189,745, 7,005,434, U.S. Publication Nos. 20070249600, 20070010534, 20060166988, 20050234083, 20050085637, 20050004142, 20040082583, 20040053943, and 20040048888, PCT Publication NOs. WO 04/009596, WO 02/060382, WO 02/039954, and WO 01/037835; [The Genomics Institute of the Novartis Research Foundation] - U.S. Publication Nos. 20070129389, and 20050222177, PCT Publication Nos. WO 08/045627, WO 08/042639, WO 07/134259, WO 07/092531, WO 07/056151, WO 07/053343, WO 07/021795, WO 07/005673, WO 06/124863, WO 06/124731, WO 06/124462, WO 06/101783, WO 06/052936, WO 05/123719, and WO 05/011597; [Scripps Research Institute] - U.S. Patent No. 7,253,166; [Aventis Pharma] - PCT Publication No. WO 06/082309; [Exelixis, Inc.] - U.S. Publication No. 20070275952, 20070161651, and 20060293342; [KYLIX, B.V.] - U.S. Publication No. 20030158199; [Merck Patent [GMBH] - U.S. Publication Nos. 20080045529, 20070293488, 20070244135, 20070225347, 20070142440, 20070112006, 20070099910, 20070066660, 20070066606, 20070021456, 20060281762, and 20060241301; [OSI Parmaceuticals] - U.S. Publication Nos. 20070129364 and 20060211678; [3-Dimensional Pharmaceuticals, Inc.] - U.S. Publication No. 20040110758; [Locus Pharmaceuticals, Inc.] - U.S. Publication No. 20070185098; [Ontogen Corp.] - U.S. Publication No. 20020183518; and [CNRS] - U.S. Publication No. 20070167519.

Xu et al., Bioorg Med Chem, 13:657-659 (2005); Semones et al., Bioorg Med Chem Lett, 17:4756-4760 (2007); Cee et al., J Med Chem, 50:627-640 (2007); Hasegawa et al., J Med Chem, 50:4453-4470 (2007); Dandu et al., Bioorg Med Chem Lett, 18:1916-1921 (2008); Becknell et al., Bioorg Med Chem Lett, 16:5368-5372 (2006); Underiner et al., Bioorg Med Chem Lett, 18:2368-2372 (2008); Miyazaki et al., Bioorg Med Chem Lett, 15:2203-2207 (2005); Kissau et al., J Med Chem, 46:2917-2031 (2003); http://www.locusdiscovery.com/programs/1p-590 (LOCUS PHARMACEUTICALS); Pyriochou et al., Br J Pharmacol, 152:1207-1214 (2007); Gingrich et al., J Med Chem, 46:5375-5388 (2003); Zhou et al., Org Lett, 3:4047-4049 (2001); Hodous et al., Bioorg Med Chem Lett, 17:2886-2889 (2007); Cao et al., J Mol Graph Model, 26:236-245 (2007); Hodous et al., J Med Chem, 50:611-626 (2007); Miyazaki et al., Bioorg Med Chem Lett, 17:1773-1778 (2007); Miyazaki et al., Bioorg Med Chem Lett, 17:250-254 (2007); White et al., Proc Natl Acad Sci USA, 100:5028-5033 (2003).

### X. Polynucleotides (Nucleic Acid Inhibitors)

In various embodiments, nucleic acid-based agents can be designed to reduce the level of endogenous ANG-2 gene expression, e.g., using antisense or ribozyme approaches to inhibit or prevent translation of ANG-2 mRNA transcripts; triple helix approaches to inhibit transcription of the ANG-2 gene; or targeted homologous recombination to inactivate or "knock out" the ANG-2 gene or its endogenous promoter.

Antisense approaches involve the design of oligonucleotides complementary to the ANG-2 polynucleotide sequence. The oligonucleotide may include other appended groups such as peptides (e.g., for targeting host cell receptors in vivo), or agents facilitating transport across the cell membrane (see, e.g., Letsinger et al., Proc Natl Acad Sci USA, 86:6553-6556 (1989); Lemaitre et al., Proc Natl Acad Sci, 84:648-652 (1987); PCT No. WO88/09810, published Dec. 15, 1988), or hybridization-triggered cleavage agents or intercalating agents (see, e.g., Zon, Pharm Res, 5:539-549 (1988)).

In one embodiment, ribozyme molecules designed to catalytically cleave mRNA transcripts having an ANG-2 polynucleotide sequence to prevent translation of ANG-2 mRNA are provided (e.g., PCT WO90/11364; U.S. Patent No. 5,824,519). As in the antisense approach, the ribozymes can be composed of modified oligonucleotides (e.g. for improved stability, targeting, and the like).

Alternatively, endogenous ANG-2 expression can be reduced by targeting deoxyribonucleotide sequences complementary to the regulatory region of the target gene (i.e., the target gene promoter and/or enhancers) to form triple helical structures that prevent transcription of the target ANG-2 gene (see generally, Helene, Anticancer Drug Des, 6:569-584 (1991); Helene et al., Ann NY Acad Sci, 660:27-36 (1992); and Maher, Bioassays, 14:807-815 (1992)).

In alternative embodiments ANG-2 expression may be blocked by post-translational gene silencing, such as by double-stranded RNA-induced gene silencing, also known as RNA interference (RNAi). RNA sequences of ANG-2 may be modified to provide double-stranded sequences or short hairpin RNAs for therapeutic use. Examples of siRNA ANG-2 inhibitors include U.S. 2004/0248174 and 2007/0134697, and PCT WO 04/094606, both of which are incorporated by reference herein for teaching these inhibitors.

### XI. Mimetics

Mimetics (e.g., "peptide mimetics" or "peptidomimetics") based upon ANG-2 or TIE2 are also provided. They may display more favorable metabolic stability, bioavailability, receptor affinity and receptor selectivity, and lower minimal side effects. Designing peptidomimetics and methods of producing the same are known in the art (see, for example, Nachman et al., Regul Pept, 57:359-370 (1995) and U.S. Pat. Nos. 6,407,059 and 6,420,118). Peptidomimetics may be derived from ANG-2, TIE2, or the extracellular domain of TIE2. These analogs can be peptides, non-peptides or combinations of peptide and non-peptide regions. Fauchere, Adv Drug Res, 15:29 (1986); Veber and Freidinger, TINS, p. 392 (1985); and Evans et al., J Med Chem, 30:1229 (1987).

In one embodiment, the ANG-2 inhibitor is a mimotope. Mimotopes and methods of production are taught in Partidos, C D, et al., Combinatorial Chem & High Throughput Screening, 5:15-27 (2002), and U.S. Patent Nos. 5,877,155 and 5,998,577, which are incorporated by reference for teaching their inhibitors.

### XII. Aptamers

In one embodiment, the ANG-2 inhibitor is an aptamer, including non-modified or chemically modified RNA, DNA, PNA, peptides, peptide-like molecules, or nucleic acid-like molecules capable of binding to ANG-2 or TIE2 with high affinity and specificity. An aptamer also can be a peptide or a nucleic acid molecule that mimics the three dimensional structure of active portions of the peptides or the nucleic acid molecules of the invention. (see, for example, James, Current Opinion in Pharmacology, 1:540-546 (2001); Colas et al., Nature, 380:548-550 (1996); Tuerk and Gold, Science, 249:505 (1990); Ellington and Szostak, Nature, 346:818 (1990)). The method of selection may be by, but is not limited to, affinity chromatography and the method of amplification by reverse transcription (RT) or polymerase chain reaction (PCR). White et al. (Proc Natl Acad Sci, 100:5028 (2003)) generated a nuclease-resistant RNA aptamer that binds and inhibits ANG-2 but not the related TIE2 agonist, ANG-1.

### XIII. Use of ANG-2 Inhibitors for Therapeutic Purposes

ANG-2 inhibitors can be used in any inflammatory condition in which monocyte adhesion to and migration across the endothelium contributes to clinical symptoms. However, certain specific embodiments of the present invention include methods for human treatment using ANG-2 inhibitors, such as those disclosed herein, in particular ANG-2 or TIE2 antibodies and peptibodies, to ameliorate or prevent (treat) a variety of inflammatory disorders in the CNS. These include brain infection, brain trauma, including traumatic brain injury (TBI), epilepsy, and chronic CNS diseases such as multiple sclerosis (MS), Alzheimer's disease (AD), and Parkinson's disease (PD). Additionally, inflammation undoubtedly contributes to other chronic CNS disorders, such as amylotropic lateral sclerosis (ALS) and Creutzfeldt-Jakob disease (CJD). The role of inflammation in CNS pathologies has been reviewed by Lucas et al, Brit J Pharmacol, 147:S232-S240 (2006).

### A. Diseases of the CNS

Epilepsy is a common neurological disorder which affects around 50 million people worldwide. Increasing evidence implicates proinflammatory cytokines in the underlying neurochemical mechanisms. Seizures and epilepsy can develop following events which induce a CNS inflammatory response, and expression of IL-1, TNFα, IL-1ra and IL-6 are increased by seizure activity.

Chronic CNS diseases are generally multifactorial, with environmental factors and genetic background contributing to the development and progression of the disease (reviewed by Campbell, Ann NY Acad Sci, 1035:117-132 (2004)). All these factors also contribute to CNS inflammation, which is further exacerbated by aging. Many of the same inflammatory mediators increase in chronic neurodegenerative diseases: activation of microglia leads to production of cytokines, superoxide radicals, NO and components of the complement system.

Multiple sclerosis (MS) is a chronic disorder in which inflammation plays a clear role. Invasion of the CNS by T cells and monocytes/macrophages leads to damage to the myelin sheaths surrounding axons, loss of neuronal function and death. Since the injured areas of the CNS vary widely, the clinical symptoms are heterogeneous, and can include fatigue, muscle weakness, areas of numbness and paralysis. Characteristically, the disease progresses in cycles of relapse, often associated with systemic infection and inflammation, and remission. Microarray analysis has revealed that many genes related to inflammatory processes are upregulated in the marginal zones of active demyelinating lesions (Mycko et al. Brain, 126:1048-57 (2003)). Environmental factors may be as important as genetic predisposition to the development of MS, and it has been proposed that the disease may be triggered in genetically susceptible individuals by viral infection (Gilden, Lancet Neurol, 4:195-202 (2005)). Many inflammatory mediators are upregulated in MS and associated with the demyelinating lesions (reviewed by Raivich & Banati, Brain Res Brain Res Rev, 46:261-81 (2004)). In animal models of the disease (usually generated by injection with myelin protein or viral infection), iNOS, complement, and the cytokines IL-1, IL-12 and TNFα are increased, changes which are also observed in MS patients and correlate with the stage of the disease.

Multiple sclerosis-related conditions include relapsing remitting multiple sclerosis, progressive-relapsing multiple sclerosis, primary progressive and secondary-progressive multiple sclerosis.

Alzheimer's disease (AD) is characterised by the progressive inability to form new memories and access existing ones, due to neuronal cell death in the hippocampus and frontal cortex. Activated microglia surround the amyloid plaques that are one of the hallmarks of the disease (Sheng et al., Neuropatho Appl Neurobiol, 24:27883 (1998)). These cells initiate a cycle of events - once activated by the amyloid peptide, Ap, they synthesize and release cytokines (IL-1, IL-6 and TNFα) and chemokines, leading to monocyte migration across the blood-brain barrier. Indeed increased levels of these cytokines have been detected in brain tissue and the cerebrospinal fluid (CSF) of patients with AD. Both TNFα and IL-1 can then increase expression of amyloid precursor protein and Ap peptide. These results suggest that CNS inflammation at least participates in amplification of the disease state. Furthermore, proteins of the complement system are associated with AD lesions, and one risk factor for AD is acute brain injury, leading to long-term inflammation, with increased expression of MHC class II, IL-1 and TNFα. Convincing evidence for a causal role of inflammation in AD comes from many studies demonstrating associations between polymorphisms in the genes encoding members of the IL-1 family and AD (e.g. Rainero et al., Neurobiol Aging, 25:1293-98 (2004)), particularly IL-1a. Statins may have protective effects in AD and other types of dementia.

Parkinson's disease (PD) is characterised by loss of dopaminergic neurons in the substantia nigra, and motor symptoms of tremor, muscle rigidity and bradykinesia. Although there are associations with mutations in the genes encoding a-synuclein and parkin, PD is otherwise sporadic, and various environmental agents including pesticides and infections, may contribute to the disease. A role of inflammation has been strongly implicated (reviewed by Gao et al., Trends Pharmacol Sci, 24:395-401 (2003)), and activated microglia are found close to degenerating substantia nigra neurons of patients with PD. Inhibiting microglial activation with agents such as naloxone, an opioid receptor antagonist, or the tetracycline minocycline is neuroprotective in animal models for PD. The complement cascade is activated, and mRNAs for complement proteins are upregulated in PD (and AD) to a greater extent than in peripheral inflammatory diseases such as rheumatoid arthritis (reviewed by McGeer & McGeer, Ann NY Acad Sci, 1035:104-116 (2004)). Some reports show that TNFα and IL-1 contribute to neuronal loss, whereas others conclude that IL-1 and IL-6, which are both elevated in the CSF of PD patients, are neuroprotective.

Amylotropic lateral sclerosis (ALS), a rapidly progressing motor neuron disease, is associated with mutation of superoxide dismutase (SOD1) gene, and mice that overexpress mutant SOD1 show upregulation of TNFα (Yoshihara et al., J Neurochem, 80:158-67 (2002)).

Creutzfeldt-Jakob disease (CJD) is associated with IL-1 levels that are elevated in cerebrospinal fluid of CJD patients, and activated microglia are detected in mice infected with CJD (Van et al., Neurobiol Aging, 23:59-64 (2002)).

Chronic psychiatric disorders may have an inflammatory component. Acute phase proteins and cytokines such as IL-1b and IL-6 are elevated in the serum of depressed patients, and IL-1ra and IFNγ are increased in bipolar disorder. The aetiology of schizophrenia remains unexplained, but recently a vascular-inflammatory-genetic theory has been proposed (Hanson & Gottesman, BMC Med Genet, 6:7 (2005)), bringing together environmental and genetic factors that influence the inflammatory response and potentially contribute to the disease. Serum levels of many cytokines are increased in schizophrenia, including IL-1b and IL-6.

Viral or protozoal infection triggers an over-stimulation of the immune system with effects in the CNS. Non-limiting examples of such infectious agents and infections are Mycoplasma pneumonia, AIDS and conditions associated with AIDS and/or related to AIDS, such as AIDS dementia complex; CMV (cytomegalvirus), bacterial or viral meningitis.

Other conditions include those resulting from injuries to the head or spinal cord, including subdural hematoma due to trauma to the head. In connection with this therapy, the ANG-2 inhibitors are suitable for preventing cranial neurologic damage and preventing and treating cervicogenic headache.

### B. Combination Therapy

The invention includes administration of an ANG-2 inhibitor to the same patient in combination with one or more additional suitable agent(s), each being administered according to a regimen suitable for that medicament. This includes concurrent administration of an ANG-2 inhibitor and one or more suitable agents or sequential administration. The term "concurrent administration" encompasses substantially simultaneous administration. "Non-concurrent" (sequential) administration encompasses administering an ANG-2 inhibitor and the additional suitable agent(s), at different times, in any order, whether overlapping or not. This includes, but is not limited to, sequential treatment (such as pretreatment, posttreatment, or overlapping treatment) with the components of the combination, as well as regimens in which the drugs are alternated, or wherein one component is administered long-term and the other(s) are administered intermittently. Components may be administered in the same or in separate compositions, and by the same or different routes of administration.

In one embodiment, the ANG-2 inhibitor may be administered with one or more anti-inflammatory agents. As used herein, the term "anti-inflammatory agent" refers generally to any agent that reduces clinical symptoms of inflammation in a patient. A number of exemplary anti-inflammatory agents are recited herein, but it will be appreciated that there may be additional suitable anti-inflammatory agents not specifically recited herein, but which are encompassed by the present invention.

The anti-inflammatory agent can be, for example, a compound that inhibits the interaction of inflammatory cytokines with their receptors. Examples of cytokine inhibitors useful in combination with the ANG-2 inhibitors of the invention include, for example, antagonists of TGF-β, IFN-α, IFN-γ, IL-6, IL8, and TNF, especially TNFα, as well as antagonists directed against other interleukins involved in inflammation. Such interleukins include, but are not limited to, IL-1, IL-2, IL-3, IL-4, IL-5, IL 6, IL 8, IL-9, IL-11, IL-12, IL-13, IL-17, and IL-18. See Feghali et al., Frontiers in Biosci, 2:12-26 (1997).

In one embodiment, a combination therapy relates to an ANG-2 inhibitor of the invention administered to a patient in combination with a TNFα inhibitor.

TNF inhibitors include, but are not limited to, receptor-binding peptide fragments of TNFα, antisense oligonucleotides or ribozymes that inhibit TNFα production, antibodies directed against TNFα, and recombinant proteins comprising all or portions of receptors for TNFα or modified variants thereof, including genetically-modified muteins, multimeric forms and sustained-release formulations. Also suitable are TACE (Tumor Necrosis Factor-α Converting Enzyme) inhibitors, such as TAPI (Immunex Corp.) and GW-3333X (Glaxo Wellcome Inc.). Also suitable are molecules that inhibit the formation of the IgA α1AT complex, such as the peptides disclosed in EP 0 614 464 B, or antibodies against this complex. Additionally suitable molecules include, but are not limited to, TNFα-inhibiting disaccharides, sulfated derivatives of glucosamine, or other similar carbohydrates described in U.S. Patent No. 6,020,323. Further suitable molecules include peptide TNFα inhibitors disclosed in U.S. Patent Nos. 5,641,751 and 5,519,000, and the D-amino acid-containing peptides described in U.S. Patent No. 5,753,628. In addition, inhibitors of TNFα converting enzyme are also suitable. WO 01/03719 describes further additional agents which can be used in combination in accordance with the invention.

ANG-2 inhibitors in combination with one or more inhibitors of IL-1, TNFα, IL-1ra, or IL-6 can be used to treat epilepsy.

ANG-2 inhibitors in combination with one or more inhibitors of IL-1, IL-12, or TNFα can be used to treat MS.

ANG-2 inhibitors in combination with one or more inhibitors of IL-1, IL-6, or TNFα can be used to treat AD.

ANG-2 inhibitors in combination with one or more inhibitors of IL-1, IL-6, or TNFα can be used to treat PD.

ANG-2 inhibitors in combination with inhibitors of TNFα can be used to treat ALS.

Still further suitable compounds include, but are not limited to, small molecules such as matrix metalloproteinase (MMP) inhibitors or other small molecules. Suitable MMP inhibitors for this purpose include, for example, those described in U.S. Patent Nos. 5,883,131, 5,863,949 and 5,861,510 as well as mercapto alkyl peptidyl compounds as described in U.S. Patent No. 5,872,146. Additional suitable small molecules include, but are not limited to, MMP inhibitors as described in U.S. Patent Nos. 5,747,514, and 5,691,382, as well as hydroxamic acid derivatives such as those described in U.S. Patent No. 5,821,262.

The present invention can also utilize an ANG-2 inhibitor and any of one or more Non-Steroidal Anti-Inflammatory Drugs (NSAIDs). NSAIDs owe their anti-inflammatory action, at least in part, to the inhibition of prostaglandin synthesis. Goodman and Gilman, The Pharmacological Basis of Therapeutics, MacMillan 7th Edition (1985). NSAIDs can be characterized into nine groups: (1) salicylic acid derivatives; (2) propionic acid derivatives; (3) acetic acid derivatives; (4) fenamic acid derivatives; (5) carboxylic acid derivatives; (6) butyric acid derivatives; (7) oxicams; (8) pyrazoles and (9) pyrazolones. Examples of NSAIDs include, but are not limited to: Anaprox™, Anaprox DSTM (naproxen sodium); Ansaid™ (flurbiprofen); Arthrotec™ (diclofenac sodium + misoprostil); Cataflam™/Voltaren™ (diclofenac potassium); Clinoril™ (sulindac); Daypro™ (oxaprozin); Disalcid™ (salsalate); Dolobid™ (diflunisal); EC Naprosyn™ (naproxen sodium); Feldene™ (piroxicam); Indocin™, Indocin SRTM (indomethacin); Lodine™, Lodine XLTM (etodolac); Motrin™ (ibuprofen); Naprelan™ (naproxen); Naprosyn™ (naproxen); Orudis™, (ketoprofen); Oruvail™ (ketoprofen); Relafen™ (nabumetone); Tolectin™, (tolmetin sodium); Trilisate™ (choline magnesium trisalicylate); Cox-1 inhibitors; Cox-2 Inhibitors such as Vioxx™ (rofecoxib); Arcoxia™ (etoricoxib), Celebrex™ (celecoxib); Mobic™ (meloxicam); Bextra™ (valdecoxib), Dynastat™ paracoxib sodium; Prexige™ (lumiracoxib), and nambumetone. Additional suitable NSAIDs, include, but are not limited to, the following: ε-acetamidocaproic acid, S-adenosylmethionine, 3-amino-4-hydroxybutyric acid, amixetrine, anitrazafen, antrafenine, bendazac, bendazac lysinate, benzydamine, beprozin, broperamole, bucolome, bufezolac, ciproquazone, cloximate, dazidamine, deboxamet, detomidine, difenpiramide, difenpyramide, difisalamine, ditazol, emorfazone, fanetizole mesylate, fenflumizole, floctafenine, flumizole, flunixin, fluproquazone, fopirtoline, fosfosal, guaimesal, guaiazolene, isonixirn, lefetamine HCl, leflunomide, lofemizole, lotifazole, lysin clonixinate, meseclazone, nabumetone, nictindole, nimesulide, orgotein, orpanoxin, oxaceprolm, oxapadol, paranyline, perisoxal, perisoxal citrate, pifoxime, piproxen, pirazolac, pirfenidone, proquazone, proxazole, thielavin B, tiflamizole, timegadine, tolectin, tolpadol, tryptamid and those designated by company code number such as 480156S, AA861, AD1590, AFP802, AFP860, AI77B, AP504, AU8001, BPPC, BW540C, CHINOIN 127, CN100, EB382, EL508, F1044, FK-506, GV3658, ITF182, KCNTEI6090, KME4, LA2851, MR714, MR897, MY309, ON03144, PR823, PV102, PV108, R830, RS2131, SCR152, SH440, SIR133, SPAS510, SQ27239, ST281, SY6001, TA60, TAI-901 (4-benzoyl-1-indancarboxylic acid), TVX2706, U60257, UR2301 and WY41770. Structurally related NSAIDs having similar analgesic and anti-inflammatory properties to the NSAIDs are also encompassed by this group.

In one embodiment, combination therapies include an ANG-2 inhibitor and the TNF inhibitor ENBREL^{®} (etanercept) for the treatment of inflammation in the CNS.

ANG-2 inhibitor compositions can be used alone or in combination with interferon β-1a (AVONEX^{®}; Biogen-Idec and REBIF^{®} EDM Serono, Inc., Pfizer, Inc.), interferon β-1b (BETASERON^{®}; Bayer Health Care.), glatiramer acetate (COPAXONE^{®}; Teva Pharmaceuticals, also as described in U.S. Publication No. 20070161566) and/or anti-VLA4 mAb (TYSABRI^{®}, Biogen-Idec, Elan).

The ANG-2 inhibitors may also be combined with one or more agents being developed to treat MS. These include Mylinax^{®} (Oral Cladribine) (Serono), Teriflunomide^{®} (leflunomide analog) (Sanofi-Aventis), Laquinomod^{®} (Active Bio/Teva), BG00012^{®} (dimethyl fumarate) (Biogen Idec/Fumapharm), Fingolimod^{®} (FTY720) (Novartis), Campath^{®} (Schering/Genzyme), Rituxan^{®} (Genentech/Roche/Biogen/Idec), and MBP8298^{®} (BioMS).

ANG-2 inhibitor compositions can be used alone or in combination with S1P agonists.

Further examples of drugs and drug types which can be administered by combination therapy include, but are not limited to, antivirals, and antibiotics, corticosteroids, antagonists of inflammatory cytokines, Disease-Modifying Anti-Rheumatic Drugs (DMARDs), Non-Steroidal Anti-Inflammatory drugs (NSAIDs), and Slow-Acting Anti-Rheumatic Drugs (SAARDs).

Exemplary Disease-Modifying Anti-Rheumatic Drugs (DMARDs) include, but are not limited to: Rheumatrex™ (methotrexate); Enbrel^{®} (etanercept); Remicade^{®} (infliximab); Humira™ (adalimumab); Segard^{®} (afelimomab); Arava™ (leflunomide); Kineret™ (anakinra); Arava™ (leflunomide); D-penicillamine; Myochrysine; Plaquenil; Ridaura™ (auranofin); Solganal; lenercept (Hoffman-La Roche); CDP870 (Celltech); CDP571 (Celltech), as well as the antibodies described in EP 0 516 785 B1, U.S. Patent No. 5,656,272, EP 0 492 448 A1; onercept (Serono; CAS reg. no. 199685-57-9); MRA (Chugai); ImuranTM (azathioprine); NFKB inhibitors; CytoxanTM (cyclophosphamide); cyclosporine; hydroxychloroquine sulfate; minocycline; sulfasalazine; and gold compounds such as oral gold, gold sodium thiomalate and aurothioglucose.

Suitable slow acting anti-rheumatic drugs (SAARDs) or disease-modifying antirheumatic drugs (DMARDS) include, but are not limited to: allocupreide sodium, auranofin, aurothioglucose, aurothioglycanide, azathioprine, brequinar sodium, bucillamine, calcium 3 aurothio-2-propanol-1-sulfonate, chlorambucil, chloroquine, clobuzarit, cuproxoline, cyclophosphamide, cyclosporin, dapsone, 15-deoxyspergualin, diacerein, glucosamine, gold salts (e.g., cycloquine gold salt, gold sodium thiomalate, gold sodium thiosulfate), hydroxychloroquine, hydroxyurea, kebuzone, levamisole, lobenzarit, melittin, 6-mercaptopurine, methotrexate, mizoribine, mycophenolate mofetil, myoral, nitrogen mustard, D penicillamine, pyridinol imidazoles such as SKNF86002 and SB203580, rapamycin, thiols, thymopoietin and vincristine. Structurally related SAARDs or DMARDs having similar analgesic and anti-inflammatory properties are also intended to be encompassed by this group.

Antimicrobials (and prodrug esters or pharmaceutically acceptable salts thereof) are also suitable for combination use as described herein. Suitable antimicrobials include, for example, ampicillin, amoxycillin, aureomicin, bacitracin, ceftazidime, ceftriaxone, cefotaxime, cephachlor, cephalexin, cephradine, ciprofloxacin, clavulanic acid, cloxacillin, dicloxacillan, erythromycin, flucloxacillan, gentamicin, gramicidin, methicillan, neomycin, oxacillan, penicillin and vancomycin. Structurally related antimicrobials having similar analgesic and anti-inflammatory properties are also intended to be encompassed by this group.

### C. Pharmaceutical Formulations, Routes Of Administration, Dosages

Pharmaceutical compositions that comprise a therapeutically effective amount of an ANG-2 inhibitor and a pharmaceutically acceptable diluent, carrier, solubilizer, emulsifier, preservative, and/or adjuvant are also provided. In addition, methods of treating a patient by administering such pharmaceutical composition are included. The term "patient" includes human patients.

In certain embodiments, acceptable formulation materials preferably are nontoxic to recipients at the dosages and concentrations employed. In certain embodiments, the pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counter ions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as Polysorbate 20, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. See, Remington's Pharmaceutical Sciences, 18th Edition, (A.R. Genrmo, ed.), 1995, Mack Publishing Company.

In certain embodiments, the optimal pharmaceutical composition will be determined by one skilled in the art depending upon, for example, the intended route of administration, delivery format and desired dosage. See, for example, *Remington's Pharmaceutical Sciences*, *supra.* In certain embodiments, such compositions may influence the physical state, stability, rate of *in vivo* release and rate of *in vivo* clearance of the antigen binding proteins disclosed. In certain embodiments, the primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. In specific embodiments, pharmaceutical compositions comprise Tris buffer of about pH 7.0-8.5, or acetate buffer of about pH 4.0-5.5, and may further include sorbitol or a suitable substitute therefor. In certain embodiments, ANG-2 inhibitors compositions may be prepared for storage by mixing the selected composition having the desired degree of purity with optional formulation agents (*Remington's Pharmaceutical Sciences*, *supra*) in the form of a lyophilized cake or an aqueous solution. Further, in certain embodiments, the human GM-CSF antigen binding protein may be formulated as a lyophilizate using appropriate excipients such as sucrose.

The pharmaceutical compositions can be selected for parenteral delivery. Alternatively, the compositions may be selected for inhalation or for delivery through the digestive tract, such as orally. Preparation of such pharmaceutically acceptable compositions is within the skill of the art.

The formulation components are present preferably in concentrations that are acceptable to the site of administration. In certain embodiments, buffers are used to maintain the composition at physiological pH or at a slightly lower pH, typically within a pH range of from about 5 to about 8.

When parenteral administration is contemplated, the therapeutic compositions may be provided in the form of a pyrogen-free, parenterally acceptable aqueous solution comprising an ANG-2 inhibitor in a pharmaceutically acceptable vehicle. A particularly suitable vehicle for parenteral injection is sterile distilled water in which the inhibitor is formulated as a sterile, isotonic solution, properly preserved. In certain embodiments, the preparation can involve the formulation of the desired molecule with an agent, such as injectable microspheres, bio-erodible particles, polymeric compounds (such as polylactic acid or polyglycolic acid), beads or liposomes, that may provide controlled or sustained release of the product which can be delivered via depot injection. In certain embodiments, hyaluronic acid may also be used; having the effect of promoting sustained duration in the circulation. In certain embodiments, implantable drug delivery devices may be used to introduce the desired antigen binding protein.

Certain pharmaceutical compositions are formulated for inhalation. In some embodiments, the inhibitors are formulated as a dry, inhalable powder. In certain embodiments, solutions may be nebulized. Pulmonary administration and formulation methods therefore are further described in PCT Publication No. WO94/20069 and describes pulmonary delivery of chemically modified proteins. Some formulations can be administered orally. Inhibitors that are administered in this fashion can be formulated with or without carriers customarily used in the compounding of solid dosage forms such as tablets and capsules. In certain embodiments, a capsule may be designed to release the active portion of the formulation at the point in the gastrointestinal tract when bioavailability is maximized and pre-systemic degradation is minimized. Additional agents can be included to facilitate absorption of the inhibitor. Diluents, flavorings, low melting point waxes, vegetable oils, lubricants, suspending agents, tablet disintegrating agents, and binders may also be employed.

Some pharmaceutical compositions comprise an effective quantity of an ANG-2 inhibitor in a mixture with non-toxic excipients that are suitable for the manufacture of tablets. By dissolving the tablets in sterile water, or another appropriate vehicle, solutions may be prepared in unit-dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional pharmaceutical compositions will be evident to those skilled in the art, including formulations involving human an ANG-2 inhibitor in sustained- or controlled-delivery formulations. Techniques for formulating a variety of other sustained- or controlled-delivery means, such as liposome carriers, bio-erodible microparticles or porous beads and depot injections, are also known to those skilled in the art. See, for example, PCT Publication No. WO 93/15722 that describes controlled release of porous polymeric microparticles for delivery of pharmaceutical compositions. Sustained-release preparations may include semipermeable polymer matrices in the form of shaped articles, e.g., films, or microcapsules. Sustained release matrices may include polyesters, hydrogels, polylactides (as disclosed in U.S. Patent No. 3,773,919 and European Patent Application Publication No. EP 058481), copolymers of L-glutamic acid and gamma ethyl-L-glutamate (Sidman et al., Biopolymers, 2:547-556 (1983)), poly (2-hydroxyethyl-inethacrylate) (Langer et al., J Biomed Mater Res, 15:167-277 (1981) and Langer, Chem Tech, 12:98-105 (1982)), ethylene vinyl acetate (Langer et al., 1981, *supra*) or poly-D(-)-3-hydroxybutyric acid (European Patent Application Publication No. EP 133,988). Sustained release compositions may also include liposomes that can be prepared by any of several methods known in the art. See, e.g., Eppstein et al., Proc Natl Acad Sci USA, 82:3688-3692 (1985); European Patent Application Publication Nos. EP 036,676; EP 088,046 and EP 143,949.

Pharmaceutical compositions used for *in vivo* administration are typically provided as sterile preparations. Sterilization can be accomplished by filtration through sterile filtration membranes. When the composition is lyophilized, sterilization using this method may be conducted either prior to or following lyophilization and reconstitution. Compositions for administration can be stored in lyophilized form or in a solution. Parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, crystal, or as a dehydrated or lyophilized powder. Such formulations may be stored either in a ready-to-use form or in a form (e.g., lyophilized) that is reconstituted prior to administration. Kits for producing a single-dose administration unit are also provided. Certain kits contain a first container having a dried protein and a second container having an aqueous formulation. In certain embodiments, kits containing single and multi-chambered pre-filled syringes (e.g., liquid syringes and lyosyringes) are provided.

The therapeutically effective amount of an ANG-2 inhibitor pharmaceutical composition to be employed will depend, for example, upon the therapeutic context and objectives. One skilled in the art will appreciate that the appropriate dosage levels for treatment will vary depending, in part, upon the molecule delivered, the indication for which the ANG-2 inhibitor is being used, the route of administration, and the size (body weight, body surface or organ size) and/or condition (the age and general health) of the patient. In certain embodiments, the clinicians may titer the dosage and modify the route of administration to obtain the optimal therapeutic effect.

A typical dosage may range from about 0.1 µg/kg to up to about 30 mg/kg or more, depending on the factors mentioned above. In specific embodiments, the dosage may range from 0.1 µg/kg up to about 30 mg/kg, optionally from 1 µg/kg up to about 30 mg/kg, optionally from 10 µg/kg up to about 10 mg/kg, optionally from about 0.1 mg/kg to 5 mg/kg, or optionally from about 0.3 mg/kg to 3 mg/kg.

Dosing frequency will depend upon the pharmacokinetic parameters of the particular ANG-2 inhibitor formulation used. Typically, a clinician administers the composition until a dosage is reached that achieves the desired effect. The composition may therefore be administered as a single dose, or as two or more doses (which may or may not contain the same amount of the desired molecule) over time, or as a continuous infusion *via* an implantation device or catheter. Appropriate dosages may be ascertained through use of appropriate dose-response data. In certain embodiments, the antigen binding proteins can be administered to patients throughout an extended time period. Chronic administration of an antigen binding protein minimizes the adverse immune or allergic response commonly associated with antigen binding proteins that are not fully human, for example an antibody raised against a human antigen in a non-human animal, for example, a non-fully human antibody or non-human antibody produced in a non-human species.

The route of administration of the pharmaceutical composition is in accord with known methods, e.g., orally, through injection by intravenous, intraperitoneal, intracerebral (intra-parenchymal), intracerebroventricular, intramuscular, intra-ocular, intraarterial, intraportal, or intralesional routes; by sustained release systems or by implantation devices. In certain embodiments, the compositions may be administered by bolus injection or continuously by infusion, or by implantation device.

The composition also may be administered locally *via* implantation of a membrane, sponge or another appropriate material onto which the desired molecule has been absorbed or encapsulated. In certain embodiments, where an implantation device is used, the device may be implanted into any suitable tissue or organ, and delivery of the desired molecule may be *via* diffusion, timed-release bolus, or continuous administration.

It also may be desirable to use the ANG-2 inhibitor pharmaceutical compositions *ex vivo.* In such instances, cells, tissues or organs that have been removed from the patient are exposed to ANG-2 inhibitor pharmaceutical compositions after which the cells, tissues and/or organs are subsequently implanted back into the patient.

In particular, an ANG-2 inhibitor can be delivered by implanting certain cells that have been genetically engineered, using methods such as those described herein, to express and secrete the polypeptide. In certain embodiments, such cells may be animal or human cells, and may be autologous, heterologous, or xenogeneic. In certain embodiments, the cells may be immortalized. In other embodiments, in order to decrease the chance of an immunological response, the cells may be encapsulated to avoid infiltration of surrounding tissues. In further embodiments, the encapsulation materials are typically biocompatible, semi-permeable polymeric enclosures or membranes that allow the release of the protein product(s) but prevent the destruction of the cells by the patient's immune system or by other detrimental factors from the surrounding tissues.

All patents and other publications identified are expressly incorporated herein by reference for the purpose of describing and disclosing, for example, the inhibitors and/or methodologies described in such publications that might be used in connection with the described. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The following examples, including the experiments conducted and the results achieved, are provided for illustrative purposes only and are not to be construed as limiting the scope of the appended claims.

### EXAMPLES

### Example 1

### Anti-ANG-2 Inhibition Reduced Monocyte Adhesion to Endothelial Cells in CNS Model of Inflammation

### Summary

The nervous system is constantly infiltrated by sentinels coming from the blood and known as perivascular macrophages. This example shows evidence that the CD68⁺GR1⁻monocytes can give rise to perivascular macrophages in mice suffering from endotoxemia. After adhesion to the endothelium, these monocytes start to crawl, adopt a rod-shaped morphology when passing through the capillaries, and can manifest the ability to proliferate and form a long cytoplasmic protuberance. They are attracted in greater numbers during endotoxemia by a combination of vasoregulatory molecules, including tumor necrosis factor, interleukin-1β, and ANG-2. After hours of delay, some of them cross the endothelium to expand the population of perivascular macrophages. A depletion of adherent monocytes and perivascular macrophages can be achieved by injection of ANG-2 peptide-Fc fusion protein. This study extends understanding of the behavior of monocytes at the blood-brain barrier and provides a way to block their infiltration into the nervous tissue during inflammatory conditions.

### Background

The central nervous system contains different populations of monocytic cells, notably microglia and perivascular macrophages. These cells have a common origin (i.e., bone marrow-derived monocytes). See, for example, Graeber et al., J Neurosci Res, 22:103-106 (1989) and Vallieres et al, J Neurosci, 23:5197-5207 (2003). Immunophenotypically, perivascular macrophages are distinguished from microglia by the presence of high levels of CD163 (ED2) and CD45 on their surface. See, for example, Graeber et al., J Neurosci Res, 22:103-106 (1989) and Sedgwick et al., Proc Natl Acad Sci USA, 88:7438-7442 (1991). They are believed to be active phagocytes involved in immune surveillance and antigen presentation, as suggested by their ability to take up foreign particles (Mato et al., Am J Anat, 172:124-140 (1985); Pennell et al., Glia, 23:84-88 (1998); Zhang et al., Acta Neuropathol (Berl), 83:233-230 (1992); Kida et al., Act Neuropathol (Berl), 85:646-652 (1993); Polfiet et al., J Neuroimmunol., 116:188-195 (2001); Bechmann et al., Exp Neurol, 168:242-249 (2001) and Bechmann et al., Eur J Neurosci, 14: 1651-1658 (2001)) and to express MHC class II and costimulatory molecules (Hickey et al., Science, 239:290-292) and Fabriek et al., Glia, 51:297-305 (2005)).

Although perivascular macrophages protect the nervous tissue against infection, injury, degenerative disease, and cancer, they can be detrimental. For example, in the pathogenesis of multiple sclerosis they present antigens to T lymphocytes and secrete proinflammatory and cytotoxic molecules. Moreover, they can provide a route of entry for viruses such as HIV, leading to encephalitis and neurological complications. It would be desirable, therefore, to selectively block the traffic of monocytes across the blood-brain barrier for therapeutic purposes. It is helpful, therefore, to understand the mechanism(s) that govern the adhesion of monocytes to the cerebral vasculature, their migration across the endothelium, and their maturation to macrophages and identify in situ the immediate precursors of cerebral macrophages.

A small population of rod-shaped leukocytes firmly attached to the luminal surface of cerebral capillaries has previously been identified (Vallieres et al., J Neurosci, 23:5197-5207 (2003)). The only information previously available about their phenotype was that they express the hematopoietic cell marker CD45, but not the macrophage marker Iba1. This example contains evidence that these cells are the precursors of perivascular macrophages and that their recruitment in the brain is enhanced during endotoxemia by a molecular cascade involving classical proinflammatory cytokines and ANG-2 (Fiedler et al., Trends Immunol, 27:552-558 (2006)).

### Results

Brain sections from chimeric mice in which ∼92% of the CD11b⁺ leukocytes expressed GFP were used to phenotype rod-shaped leukocytes found in the cerebral vasculature. A majority of GFP⁺ rod-shaped leukocytes expressed the myeloid marker CD11b and the monocytic marker CD68, while a minority expressed the granulocytic marker GR1. The nucleus of the CD68⁺ cells was monolobed, whereas that of the GR1⁺ cells was multilobed. Rod-shaped leukocytes expressed the monocytic markers F4/80, CD163, and stabilin-1. No cell positive for the lymphocyte marker CD3 was detected. Overall, these results indicate that the population of rod-shaped leukocytes is a mixture of monocytes and granulocytes. These cells were comparable to the round leukocytes also found attached to the brain vasculature except for their morphology and the fact that they were twice more numerous, as reported previously (Vallieres et al., J Neurosci, 23:-5197-5207 (2003)).

Most rod-shaped leukocytes exhibited morphological features typical of migrating cells, such as a leading edge (front of the cell) and uropod (rear of the cell). Organotypic brain slice cultures were examined by time-lapse microscopy. Rod-shaped leukocytes in the process of migration were observed. This result, together with the observation that most rod-shaped leukocytes have a polarized morphology, suggests that these cells do not stay in place after adhesion, but move by crawling on the endothelial surface.

To examine whether the number of rod-shaped leukocytes increases during inflammation, normal mice (nonirradiated) were injected intraperitoneally with bacterial LPS, to induce a systemic inflammatory response that involves the activation of cerebral macrophages (48). Brain sections from mice killed at different time points were stained for CD45 to reveal all immune cells. The number of rod-shaped leukocytes was about four times higher in the cerebral cortex of mice killed 6 or 12 h after LPS injection compared to saline-treated mice. A proportional increase in the number of round leukocytes was observed. The ratio of CD68⁺ monocytes to GR1⁺ granulocytes was roughly 3:1 and did not vary significantly according to the treatment.

As was previously reported (Vallieres et al, J Neurosci, 23:5197-5207 (2003)), a small proportion of rod-shaped leukocytes was observed, generally located at capillary branch points, that were apparently in division. This subpopulation included CD68⁺ monocytes and GR1⁺ granulocytes. These cells have the potential to proliferate. Using BrdU to label cells in the S phase of the cell cycle revealed the presence of rodshaped leukocytes that had incorporated BrdU. This confirms that these cells can proliferate while they are in the vasculature.

The proliferation of rod-shaped leukocytes increases during endotoxemia. The percentage of CD45⁺ rod-shaped cells in process of division in the cerebral cortex of mice killed 6 h after injection of LPS or saline into the peritoneum was examined. This percentage was almost twice higher in LPS-treated mice. This suggests that the number of rod-shaped leukocytes is controlled not only at the level of adhesion, but also, to some extent, at the level of proliferation.

Rod-shaped leukocytes could patrol the cerebral vasculature to be ready to intervene quickly when and where needed by entering the parenchyma and differentiating into macrophages. This possibility was examined. Evidence for the ability of rod-shaped leukocytes to cross the blood-brain barrier in chimeric mice suffering from endotoxemia was found. One day after LPS injection GFP⁺ rod-shaped leukocytes were observed that seemed in process of transmigration. Others were clearly inside the parenchyma and exhibited a polarized morphology typical of migrating cells. In contrast, no round leukocytes were found in the parenchyma. All the infiltrated rod-shaped leukocytes had a monolobed nucleus indicative of monocytic lineage. These cells shared with perivascular macrophages many characteristics, including an elongated morphology and the expression of GFP, CD11b, CD68, CD163, and stabilin-1. As reported previously (Vallieres, J Neurosci, 23:5197-5207 (2003)), the only differences observed between rod-shaped monocytes and perivascular macrophages were that the latter expressed Iba1 and had a more elongated and irregular cytoplasm. In this regard, some rod-shaped monocytes manifested the ability to extend a long cytoplasmic process while they were inside the vasculature. These cells, as those located in the parenchyma, were only observed 24 h after LPS injection and not at the other time points examined (6 h and 3 days) and in a steady state.

After entering the brain, rod-shaped monocytes presumably have two options: differentiate into microglia or perivascular macrophages. Accordingly, if the traffic of rod-shaped monocytes across the endothelium is accelerated during endotoxemia, an increase in the formation of either one or both of these cells would be expected. To examine this possibility, the number of GFP⁺ cells of different subsets (round cells, rod-shaped cells, perivascular macrophages, and microglia) in the cerebral cortex of chimeric mice killed 1 or 3 days after intraperitoneal LPS injection was assessed. Stereological analysis revealed an increase (67%) of GFP⁺ perivascular macrophages, but only at 3 days post-injection. By comparison, the number of intravascular leukocytes was increased at both time points, whereas that of microglia was unchanged. Altogether, these results suggest that the formation of perivascular macrophages is increased in response to endotoxemia (relative to non-endotoxemic conditions), at least in part, via the recruitment of rod-shaped monocytes.

Many of the effects of LPS are mediated by proinflammatory molecules, especially TNF and IL-1ß. To determine the importance of these two cytokines in the control of the number of rod-shaped leukocytes, LPS was injected into mice deficient or not in TNF and/or IL-1ß. Their brains were examined 6 h later for histological analysis. As previously reported, LPS strongly induced the expression of both cytokines throughout the brains of wild-type mice, particularly along blood vessels. Brain sections were stained for CD45. The number of rod-shaped leukocytes was ∼62% lower in all the knockouts compared to LPS-treated wild-types. A proportional decrease in round leukocytes was detected. The effect of LPS was not totally abolished in the knockouts, as the number of rod-shaped leukocytes was about twice that found in saline-treated wild-types. Furthermore, no difference was detected between the single and double knockouts, indicating that TNF and IL-1ß are both essential for an optimal response to LPS, but that they do not act in an additive or synergistic manner.

Proinflammatory molecules promote leukocyte adhesion and transmigration by inducing vascular changes (Muller, Trends Immunol, 24:327-334 (2003) and Imhof et al., Nat Rev Immunol, 4:432-444 (2004)). To identify endothelial factors induced in the brain during endotoxemia and potentially involved in the recruitment of rod-shaped leukocytes, RNA samples that were extracted from the brains of mice killed 6 h after LPS injection were analyzed using oligonucleotide microarrays .

Only upregulated genes with a P value < 0.05 and classified as extracellular or plasma membrane-associated by the Gene Ontology Consortium were retained. Among the 76 remaining genes, many were known to be inducible in endothelial cells, including cytokine receptors (e.g., TNF receptor 1, IL-1 receptor 1), adhesion molecules (e.g., ICAM1, VCAM1), and ANG-2.

To confirm that LPS can induce ANG-2 expression, brain sections from mice killed at different time points after intraperitoneal injection of LPS or saline were analyzed for the presence of ANG-2 mRNA by radioisotopic in situ hybridization. Virtually no signal was detected in normal conditions and 1 h after LPS injection (data not shown). In contrast, strong signals were observed throughout the brain from 3 to 12 h, with a maximum number at 6 h. No more signal was detected after 24 h. Consistent with these results, real-time PCR analysis revealed a 3.4-fold increase in the levels of ANG-2 mRNA in the brains of mice killed 6 h post-LPS injection compared to saline-treated mice, but no difference in mice killed after 24 h. To determine the identity of ANG-2-expressing cells, adjacent sections were double labeled for ANG-2 mRNA and different cell type-specific markers by combined in situ hybridization and immunohistochemistry. In a vast majority hybridization signals colocalized with the endothelial marker CD31 and very rarely (-3 per section) with the astrocytic marker GFAP. No colocalization was observed with the macrophage marker Iba1. Overall, these results indicate that ANG-2 is expressed during endotoxemia in a transient manner almost exclusively by the endothelium.

To determine whether TNF and IL-1ß mediate the effect of LPS on ANG-2 expression, in situ hybridization was assessed to analyze the brains of mice deficient or not in either one or both of these cytokines and killed 6 h after LPS injection. There was a 43% decrease in the number of ANG-2 mRNA⁺ cells in the cerebral cortex of TNF-deficient mice compared to wild-type mice. A similar decrease was observed in mice lacking both TNF and IL-1ß, but not in mice lacking only IL-1ß. These results suggest that TNF, but not IL-1ß, mediates in part the effect of LPS on ANG-2 expression.

Brain sections from mice killed 3 or 6 h after intracerebral injection of TNF or saline were analyzed by in situ hybridization to confirm the ability of TNF to induce ANG-2 expression. Strong signals were found only around the needle track in control mice, but in a large proportion of the ipsilateral hemisphere in TNF-treated animals. More precisely, stereological analysis revealed that the volume occupied by ANG-2 mRNA⁺ cells was 6.5 times larger in mice killed 6 h after TNF injection compared to saline-treated mice.

Mouse cerebral endothelial cells were cultured for 6 h in the presence of TNF, LPS, and/or anti-TNF antibody to study in vitro the regulation of ANG-2 expression. Real-time PCR analysis revealed that TNF and LPS induced a more than 2-fold increase in the levels of ANG-2 mRNA. The induction by TNF, but not by LPS, was blocked in the presence of anti-TNF antibody, confirming the specificity of the result. For comparison, astrocytes were also analyzed. These cells were found to also express ANG-2 mRNA in basal culture conditions. Contrary to what was found in endothelial cells, the levels of ANG-2 mRNA in astrocytes were decreased after exposure to TNF or LPS. Moreover, the effect of LPS and not only of TNF was blocked by coincubation with anti-TNF antibody. Overall, these results indicate that ANG-2 expression is regulated differentially by LPS depending on the cell type: it is induced in endothelial cells by a mechanism that can be independent of TNF, whereas it is inhibited in astrocytes by a TNF-dependent mechanism. In agreement with this, there was a 264-fold increase in the levels of TNF mRNA in LPS-treated astrocytes compared to saline treated astrocytes, but no significant expression of TNF in endothelial cells. The detection of TNF mRNA in astrocytes was not due to contaminating cells as the cultures were >99% pure, as estimated by GFAP staining.

To investigate the role of ANG-2 in the recruitment of rod-shaped leukocytes, two experiments were performed in which mice were treated with the anti-ANG-2 peptide-Fc fusion protein L1-10 (L1-10 is disclosed in Oliner et al., Cancer Cell, 6:507-516 (2004)). In the first experiment, normal mice were implanted with an intracerebral cannula, through which L1-10 or PBS was infused for a week, and then challenged for 6 h with LPS. In this approach, it was assumed that L1-10 would neutralize ANG-2 derived from both sides of the blood-brain barrier after diffusion through the parenchyma and absorption into the plasma. Stereological analysis revealed that the number of CD45⁺ intravascular leukocytes was decreased by 44% in mice receiving L1-10.

In the second experiment, chimeric mice were injected intravenously with L1-10 12 h before and after LPS injection and then killed three days later. This approach was used to examine not only the effect of L1-10 on leukocyte adhesion when administrated via a different route, but also its effect on the formation of perivascular macrophages. There was a 40% decrease of GFP+ intravascular leukocytes and a 51% decrease of GFP⁺ perivascular macrophages in mice treated with L1-10, but no difference in GFP⁺ microglia. Similar results were obtained by counting the number of CD45⁺ cells. Therefore, the results of these two experiments demonstrate that LPS increases the population of perivascular macrophages via the recruitment of circulating monocytes by a mechanism involving ANG-2. This molecule influences the adhesion of leukocytes and not their proliferation, as no intergroup difference in the proportion of dividing rod-shaped leukocytes was observed in both experiments.

### Conclusion

The results presented in this Example show that perivascular macrophages derive from CD68⁺GR1⁻ rod-shaped monocytes that crawl on the endothelium. These cells are attracted in greater numbers during endotoxemia by a TNF/ANG-2-dependent mechanism. While still in the vasculature, they can self-renew and monitor their environment by extending a relatively long cytoplasmic process. After a delay of several hours, some of these cells cross the endothelium and differentiate into perivascular macrophages. These observations extend our understanding of the behavior of monocytes at the blood-brain interface and the molecular mechanism that control their recruitment during inflammation. The immune response described here may be important not only in infection, but also in other conditions in which proinflammatory cytokines are produced and monocytes are recruited, such as injury, degenerative disease, cancer, and chronic neuroinflammatory disease.

### Discussion

Originally considered as a homogeneous population, circulating monocytes are now divided into two subsets: the so-called "inflammatory" (type 1) monocytes, which are selectively recruited into inflamed tissues, and the "resident" (type 2) monocytes, which home to most normal tissues (Geissmann et al., Immunity, 19:71-82 (2003) and Sunderkotter et al., J Immunol, 172:4410-4417 (2004)). These cells are respectively GR1⁺CCR2⁺CX3CR1low and GR1⁻CCR2⁻CX3CR1high. In the brain, the ability of type 1 monocytes to differentiate into microglia after injury has recently been demonstrated (Mildner et al., Nat Neurosci, 10:1544-1553 (2007)), but the fate of type 2 monocytes was so far unknown. The results in this Example show that monocytes crawling on the cerebral endothelium are all of type 2 (GR1⁻) and can give rise to perivascular macrophages.

The observation that type 2 monocytes are selectively and rapidly recruited in response to classical inflammatory stimuli (LPS, TNF, IL-1ß) is somehow surprising in the light of previous studies showing a preferential recruitment of type 1 monocytes into inflamed sites (e.g., adjuvant-treated skin, infected peritoneum, injured myocardium, atherosclerotic plaques (Geissmann et al., Immunity, 19:71-82 (2003); Sunderkotter et al., J Immunol, 172:4410-4417 (2004); Jutila et al., European Journal of Immunology, 18:1819-1826 (1988); Chan et al., Blood, 92:1423-1431 (1998); Robben et al., JExp Med, 201:1761-1769 (2005); LeBorgne et al., Immunity, 24:191-201 (2006); Nahrendort et al., J Exp Med, 204:3037-3047 (2007); and Tacke et al., J Clin Invest, 117:185-194 (2007))).

Taken together, the data indicate that LPS increases the number of crawling monocytes in the cerebral vasculature by inducing ANG-2 expression in endothelial cells, both directly and indirectly via TNF.

Contrary to the general belief that monocytes circulating in the blood are noncycling (Geissmann et al., Immunity, 19:71-82 (2003)), the results show that these cells can undergo division after adhesion to capillaries, suggesting that endothelial cells provide a proliferative signal to monocytes as they do for other cell types (e.g., neural stem cells (Shen et al., Science, 304:1338-1340 (2004))).

This study is the first to show that the population of perivascular macrophages can be expanded in response to immune challenge. This expansion results from the recruitment of circulating monocytes, although the proliferation of pre-existing macrophages could also contribute, as suggested by the previous observation that these cells can incorporate BrdU (Vallieres et al., J Neurosci, 23:5197-5207 (2003)). However, the proliferation of perivascular macrophages is minor in the model studied here, because these cells were generally not distributed in clusters, as expected if they proliferate, but rather individually. The infiltration of monocytes into the perivascular space is not a consequence of irradiation, as suggested for microglia (Mildner et al, Nat Neurosci, 10:1544-1553 (2007)), because it has been shown that monocytes, labeled ex vivo and injected into the circulation of normal mice, can give rise to perivascular macrophages (Bechmann et al, Exp Neurol, 168:242-249 (2001).

While it is widely believed that the main source of ANG-2 is the endothelium, the results show that astrocytes can produce this molecule in vivo and in vitro.

In summary, the study identifies CD68⁺GR1⁻ rod-shaped monocytes as the immediate precursors of perivascular macrophages and reveals a molecular mechanism acting on the endothelium that controls their recruitment during systemic inflammation. The observations do not preclude a role for type 2 monocytes in the production of microglia and other populations of cerebral macrophages (i.e., those of the meninges and circumventricular organs).

### Example 2

### Treatment in Animal Model of Multiple Sclerosis Using ANG-2 Peptide and Antibody Inhibitors

### Evaluation of anti-ANG2 monoclonal antibody H4L4 and anti-ANG2 peptibody L1-7 in the SJL/PLP₁₃₉₋₁₅₁ Experimental Autoimmune Encephalomyelitis model.

Experimental Autoimmune Encephalomyelitis (EAE), a preclinical model of multiple sclerosis, was induced in 11-week old female SJL/J mice by immunization with PLP₁₃₉₋₁₅₁, a peptide of proteolipid protein (PLP) which is a component of myelin. Complete Freund's adjuvant was prepared by mixing 10 ml incomplete Freund's adjuvant plus 30mg *M*. *tuberculosis* H37Ra (final conc. = 3 mg/mL *M. tuberculosis).* A 1:1 emulsion of PLP₁₃₉₋₁₅₁ and CFA was made by mixing equal volumes of 3 mg/mL PLP₁₃₉₋₁₅₁ with CFA. The emulsion was drawn into a 1-cc glass tuberculin syringe. Mice were injected subcutaneously (SC) with a total of 200 mL of emulsion containing 300 µg PLP and 300 µg *M. tuberculosis* H37 Ra.

On the day of immunization (Day 0) groups of mice were given 2 mg anti-ANG2 mAb H4L4 (n=14) or 2 mg anti-streptavidin IgG2 isotype control (N=15) intraperitoneally (IP), or 6 mg anti-ANG2 Pb L1-7 (n=15) or 6 mg human Fc isotype control subcutaneously (SC). Mice were monitored, weighed, and scored for clinical disease every other day until disease was observed, then daily thereafter.

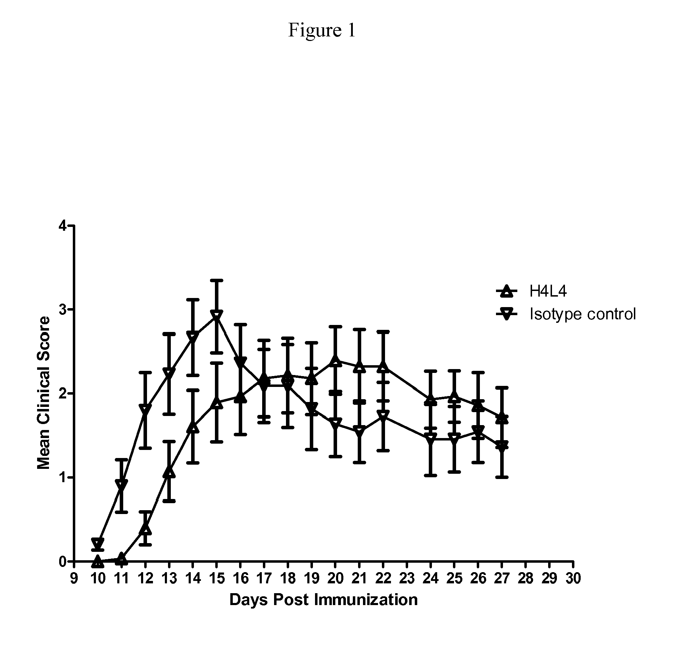

Scoring of clinical signs of disease was as follows: 0, no disease; 1, limp tail; 2, impairment of righting reflex or abnormal gait; 3, severe hind-limb weakness, partial hind-limb paralysis; 4, complete hind-limb paralysis, mobile using forelimbs. Treatment with anti-ANG2 mAb H4L4 reduced mean clinical score compared to isotype control on days 10-12 post-immunization (p<0.05, One way ANOVA with Tukey's post hoc test, Figure 1).

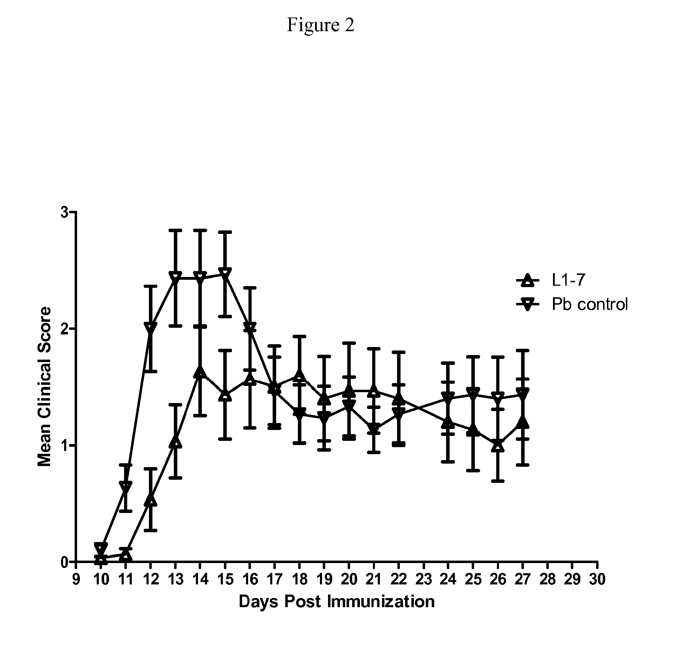

Treatment with anti-ANG2 Pb L1-7 reduced mean clinical score compared to the human Fc control on days 12-13 post-immunization (p<0.05, One way ANOVA with Tukey's post hoc test, Figure 2). Four of 15 mice in the human IgG2 isotype control group developed severe disease that necessitated euthanasia. This was not observed in either the anti-ANG2 mAb H4L4 or the anti-ANG2 Pb L1-7 treatment groups.

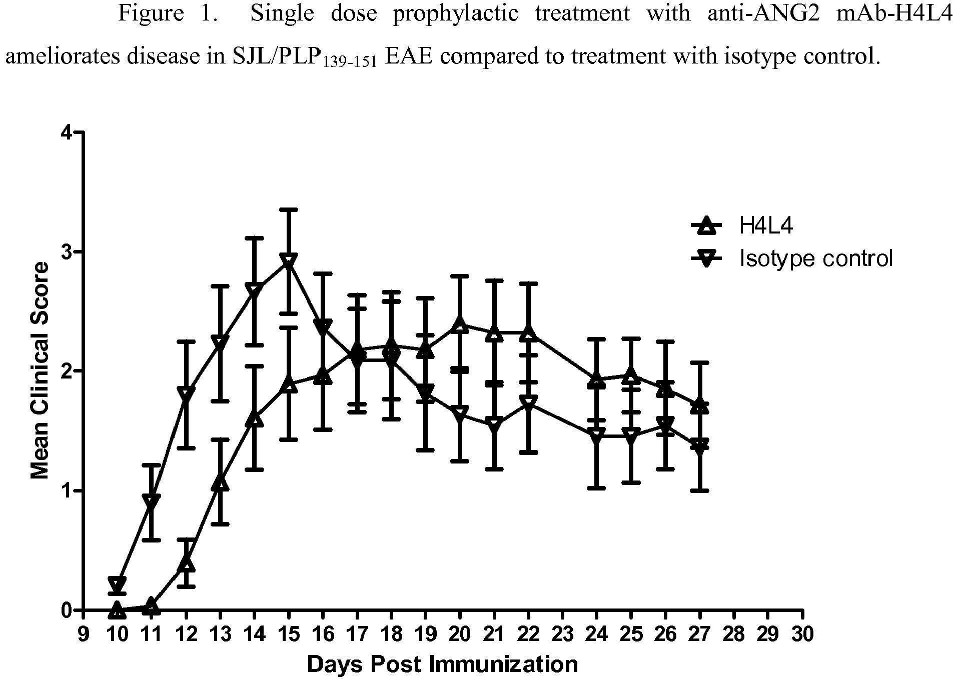

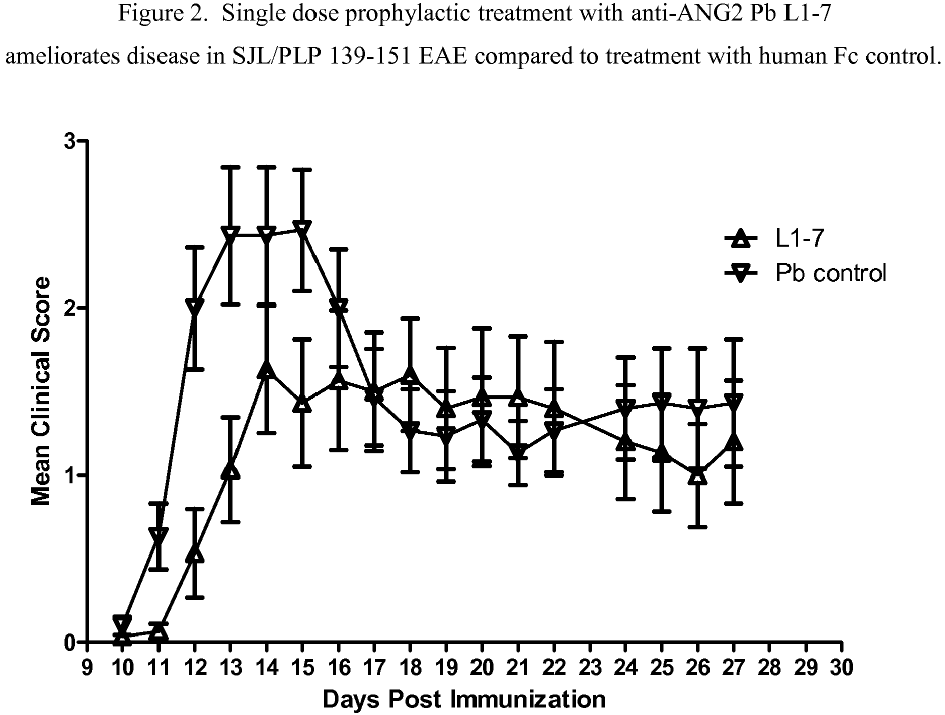

### Analysis of Results

All analysis was performed using GraphPad Prism 5.

Disease onset and incidence: A Kaplin Meier graph was used to plot the data and calculate mean onset and incidence. Significance was determined using a One Way ANOVA test with a Tukey's post hoc test.

Mean Clinical Score: Mean clinical score ± SD was graphed using an XY line graph. Significance was determined using a One Way ANOVA test with a Tukey's post hoc test.

Mean Weight: Mean weight ± SD was graphed using an XY line graph. Significance was determined using a One Way ANOVA test with a Tukey's post hoc test.

Figure 3A - Amino Acid Sequence of Human TIE2

TIE2 is 1124 a (Genbank)

mdslaslvlc gvslllsgtv egamdlilin slplvsdaet sltciasgwr phepitigrd
fealmnqhqd plevtqdvtr ewakkvvwkr ekaskingay fcegrvrgea irirtmkmrq
qasflpatlt mtvdkgdnvn isfkkvlike edaviykngs fihsvprhev pdilevhlph
aqpqdagvys aryiggnlft saftrlivrr ceaqkwgpec nhlctacmnn gvchedtgec
icppgfmgrt cekacelhtf grtckercsg qegcksyvfc lpdpygcsca tgwkglqcne
achpgfygpd cklrcscnng emcdrfqgcl cspgwqglqc eregiprmtp kivdlpdhie
vnsgkfnpic kasgwplptn eemtlvkpdg tvlhpkdfnh tdhfsvaift ihrilppdsg
vwvcsvntva gmvekpfnis vkvlpkplna pnvidtghnf avinissepy fgdgpikskk
llykpvnhye awqhiqvtne ivtlnylepr teyelcvqlv rrgeggeghp gpvrrfttas
iglppprgln llpksqttln ltwqpifpss eddfyvever rsvqksdqqn ikvpgnltsv
llnnlhpreq yvvrarvntk aqgewsedlt awtlsdilpp qpenikisni thssaviswt
ildgysissi tirykvqgkn edqhvdvkik natitqyqlk glepetayqv difaennigs
snpafshelv tlpesqapad lgggkmllia ilgsagmtcl tvllafliil qlkranvqrr
maqafqnvre epavqfnsgt lalnrkvknn pdptiypvld wndikfqdvi gegnfgqvlk
arikkdglrm daaikrmkey askddhrdfa gelevlcklg hhpniinllg acehrgylyl
aieyaphgnl ldflrksrvl etdpafaian stastlssqq llhfaadvar gmdylsqkqf
ihrdlaarni lvgenyvaki adfglsrgqe vyvkktmgrl pvrwmaiesl nysvyttnsd
vwsygvllwe ivslggtpyc gmtcaelyek lpqgyrlekp lncddevydl mrqcwrekpy
erpsfaqilv slnrmleerk tyvnttlyek ftyagidcsa eeaa

Figure 3B - Putative Extracellular, Transmembrance, and Cytoplastic Domains in TIE2.

20070280947 - Human Tie-2 (Swiss Prot database Accession No. Q02763, incorporated herein by reference), which has a similar structural organization as Tie-1, comprises an amino acid sequence of 1124 amino acids, of which about residues 1-22 comprise a signal peptide and residues 746-770 comprise the putative transmembrane domain.
SwissProt:

| SIGNAL PEPTIDE | | 1 | 22 | 22 |
|---|---|---|---|---|
| CHAIN | Angiopoietin-1 receptor /FTId=PRO_0000024474 | 23 | 1124 | 1102 |
| TOPOLOGICAL DOMAIN | Extracellular (POTENTIAL) | 23 | 745 | 723 |
| TRANSMEMBRANE REGION | POTENTIAL | 746 | 770 | 25 |
| TOPOLOGICAL DOMAIN | Cytoplasmic (POTENTIAL) | 771 | 1124 | 354 |

Figure 3C - Amino Acid Sequence of Human ANG-2

Polypeptide set forth in Figure 6 of U.S. Patent No. 6,166,185 ("Tie-2 ligand-2") ((SEQ ID NO: 6)) 496 aa (GenBank) accession no. NP_001138

mwqivfftls cdlvlaaayn nfrksmdsig kkqyqvqhgs csytfllpem dncrsssspy
vsnavqrdap leyddsvqrl qvlenimenn tqwlmkleny iqdnmkkemv eiqqnavqnq
tavmieigtn llnqtaeqtr kltdveaqvl nqttrlelql lehslstnkl ekqildqtse
inklqdknsf lekkvlamed khiiqlqsik eekdqlqvlv skqnsiieel ekkivtatvn
nsvlqkqqhd lmetvnnllt mmstsnsakd ptvakeeqis frdcaevfks ghttngiytl
tfpnsteeik aycdmeaggg gwtiiqrred gsvdfqrtwk eykvgfgnps geywlgnefv
sqltnqqryv lkihlkdweg neayslyehf ylsseelnyr ihlkgltgta gkissisqpg
ndfstkdgdn dkcickcsqm ltggwwfdac gpsnlngmyy pqrqntnkfn gikwyywkgs
gyslkattmm irpadf

Figure 3D - Amino Acid Sequence of Human ANG-1

498 aa (GenBank) accession no. NP_001137

mtvflsfafl aailthigcs nqrrspensg rrynriqhgq caytfilpeh dgncresttd

qyntnalqrd aphvepdfss qklqhlehvm enytqwlqkl enyivenmks emaqiqqnav

qnhtatmlei gtsllsqtae qtrkltdvet qvlnqtsrle iqllenslst yklekqllqq

tneilkihek nsllehkile megkhkeeld tlkeekenlq glvtrqtyii qelekqlnra

ttnnsvlqkq qlelmdtvhn lvnlctkegv llkggkreee kpfrdcadvy qagfnksgiy

tiyinnmpep kkvfcnmdvn gggwtviqhr edgsldfqrg wkeykmgfgn psgeywlgne

fifaitsqrq ymlrielmdw egnraysqyd rfhignekqn yrlylkghtg tagkqsslil

hgadfstkda dndncmckca lmltggwwfd acgpsnlngm fytagqnhgk lngikwhyfk

gpsyslrstt mmirpldf

## Claims

1. An ANG-2 inhibitor for use in treating multiple sclerosis in a human subject wherein said ANG-2 inhibitor is a 2XCon4(C) peptibody (SEQ ID NO: 56).

2. The ANG-2 inhibitor for use according to claim1, wherein said inhibitor is PEGylated.

3. The ANG-2 inhibitor for use according to claim 1 or 2, wherein said inhibitor is glycosylated.

4. The ANG-2 inhibitor for use according to anyone of claims 1 to 3, wherein said inhibitor is formulated in a solution, suspension, or lyophilized powder.

5. The ANG-2 inhibitor for use according to claim 4, wherein said inhibitor is in a pre-filled syringe.

6. A pharmaceutical composition comprising an ANG-2 inhibitor as defined in anyone of claims 1 to 4 and a pharmaceutically acceptable carrier for use in treating multiple sclerosis in a human subject.

7. An ANG 2 inhibitor as defined in anyone of claims 1 to 5 or a pharmaceutical composition as defined in claim 6 in combination with an anti inflammatory agent, for use in treating multiple sclerosis in a human subject.

8. An ANG 2 inhibitor or pharmaceutical composition and an anti inflammatory agent for use according to claim 7, wherein the anti inflammatory agent is selected from the group consisting of a DMARD, a SAARD, an NSAID, methotrexate, a TNF inhibitor, an IL-1 inhibitor, a TACE inhibitor, a COX-2 inhibitor and a P-38 inhibitor.

9. The ANG-2 inhibitor or pharmaceutical composition for use according to claim 8, whrein the TNF inhibitor comprises one or more of etanercept, adalimumab, pegsunercept sTNF-R1, onercept and infliximab.

10. The ANG-2 inhibitor or pharmaceutical composition for use according to claim 8, wherein the IL-1 inhibitor is anakinra, IL-1 TRAP, IL-1 antibody or soluble IL-1 receptor.
